(19) **Europäisches Patentamt / European Patent Office / Office européen des brevets**

(11) **EP 2 942 621 B1**

(12) **FASCICULE DE BREVET EUROPEEN**

(45) Date de publication et mention
de la délivrance du brevet:
**14.04.2021 Bulletin 2021/15**

(51) Int Cl.:
*G01N 33/24* *(2006.01)*    *A01C 21/00* *(2006.01)*
*C12Q 1/02* *(2006.01)*    *C12Q 1/12* *(2006.01)*

(21) Numéro de dépôt: **15290111.2**

(22) Date de dépôt: **28.04.2015**

(54) **DIAGNOSTIC DE L'ÉTAT DIAZOTROPHE DE SOLS ARABLES ET PRÉCONISATION DES APPORTS D'ENGRAIS AZOTÉ**

DIAGNOSE DES STICKSTOFFBINDENDEN ZUSTANDS VON ACKERBÖDEN, UND EMPFEHLUNG DES EINZUBRINGENDEN STICKSTOFF DÜNGERS

DIAGNOSIS OF THE DIAZOTROPHIC STATE OF ARABLE SOIL AND RECOMMENDATION FOR THE ADDITION OF NITROGEN FERTILISER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.05.2014 FR 1401033**

(43) Date de publication de la demande:
**11.11.2015 Bulletin 2015/46**

(73) Titulaire: **Polyor SARL**
**54000 Nancy (FR)**

(72) Inventeur: **Claude, Pierre-Philippe**
**54000 Nancy (FR)**

(56) Documents cités:
**EP-A1- 2 647 612    US-A1- 2007 186 830**

- **DESLIPPE J R ET AL: "Impacts of warming and fertilization on nitrogen-fixing microbial communities in the Canadian High Arctic", FEMS MICROBIOLOGY ECOLOGY, ELSEVIER, NL, vol. 53, no. 1, 1 juin 2005 (2005-06-01), pages 41-50, XP027780054, ISSN: 0168-6496 [extrait le 2005-06-01]**
- **DUMONT B ET AL: "Parameter identification of the STICS crop model, using an accelerated formal MCMC approach", ENVIRONMENTAL MODELLING AND SOFTWARE, vol. 52, 27 décembre 2004 (2004-12-27), pages 121-135, XP028802942, ISSN: 1364-8152, DOI: 10.1016/J.ENVSOFT.2013.10.022**
- **JEGO G ET AL: "Predicting soil water and mineral nitrogen contents with the STICS model for estimating nitrate leaching under agricultural fields", AGRICULTURAL WATER MANAGEMENT, ELSEVIER, AMSTERDAM, NL, vol. 107, 11 January 2012 (2012-01-11), pages 54-65, XP028466168, ISSN: 0378-3774, DOI: 10.1016/J.AGWAT.2012.01.007 [retrieved on 2012-01-20]**

Printed by Jouve, 75001 PARIS (FR)

**Description**

**DOMAINE TECHNIQUE DE L'INVENTION**

Agronomie, fertilisation des cultures agronomiques, et microbiologie des sols applicable aux dynamiques carboné et azoté, notamment en présence de résidus de culture cellulosiques. Il est aussi question d'informatique et de modélisation dynamique de processus sol/plante, notamment en présence de grandes cultures agronomiques.

**ÉTAT DE LA TECHNIQUE**

Le pilotage de la fertilisation azotée des grandes cultures nécessite le calcul préalable d'une certaine dose d'engrais N (azotés), dite "dose X", ou ici dX Ce calcul a traditionnellement impliqué une estimation des fournitures en azote provenant de la minéralisation de la matière organique et des résidus de culture cellulosiques au sol (RCsol) (*cf. Jego et al.* 2012). En présence de divers types de *biofertilisations*, notamment azotobactériennes (AZB) de ces RCsol, cette dX est parfois réduite, *rationnée* en sorte.

Or, la fertilisation raisonnée de l'azote avec pilotage intra - saisonnier (FRANPIS), particulièrement importante lors d'AZB des RCsol et/ou dudit rationnement de la dX, est à ce jour mal perçue par les utilisateurs. En effet, FRANPIS implique le rationnement de la dX de l'ordre de 20 à 40 UN, ration que ne sera rapportée *in fine* que si une certaine mesure de l'état azotée de la culture le préconise ; dans le cas contraire l'utilisateur peut en faire l'impasse. Cela dit, cette impasse est parfois perçue comme pouvant se faire au détriment d'un plein rendement protéique ; c'est en partie ce risque perçu qui rend la FRANPIS impopulaire chez les utilisateurs.

Faute d'une telle FRANPIS, en présence d'AZB des RCsol on opte souvent pour un simple rationnement de la dX de l'ordre d'une vingtaine unités d'azote. L'utilisateur de *biofertilisations* AZB devrait donc être en mesure de décider, sans recourir à la FRANPIS, si ce rationnement de la dX est praticable *sans* réduction du potentiel de rendement de la culture et/ou de perte d'efficacité d'AZB ; cela n'est pas le cas aujourd'hui. En effet, le rationnement, aveugle en sorte, de la dX, paradoxalement, nuie parfois à l'efficacité de telles biofertilisations AZB (*cf.* Claude et Fillion 2004 ; EP2223586A1, FR0900867), et provoque ainsi une certaine baisse, limitée mais néanmoins sensible, des teneurs en protéines des grains céréaliers à la récolte.

Il serait donc envisageable d'adapter le calcul, ou plus exactement le rationnement de la dX lors de ce type de biofertilisation AZB. En effet, au cours de l'inter - culture hivernale, d'octobre à la fin février de l'année suivante par exemple, cette AZB pourrait avoir contribuer à augmenter, à "gonfler" en sorte, les réserves d'N d'origine diazotrophique (Nif) ; une fois relargué et minéralisé in situ, cet azote pourra(it) contribuer à l'alimentation en N de la culture. Encore faut-il pouvoir évaluer, objectivement et plus simplement par rapport à l'actuelle FRANPIS, l'ampleur de ce "gonflement diazotrophe" avant d'opter, ou non, pour un quelconque rationnement de la dX d'engrais N.

Sigles et définitions

**AZB** : azotobactérisation, généralement ici des résidus de culture cellulosique au sol (RCsol) - eg. Claude et Fillon 2004.

**biofertilisation** : apports d'actifs, chimiques, minéraux, organiques et/ou microbiologiques, peu importe, capable d'avoir une effet pondéral sur la production de biomasse et/ou le rendement, disproportionnée par rapport à sa masse ; le terme anglo-saxon de "onput" par opposition à "input" est en ce sens révélateur. L'apport de microorganismes par inoculation ayant été précurseur, biofertilisation est depuis souvent compris comme synonyme de biofertilisation.

**IDM** : indice diazoto - molaire - l'IDM est constitué d'une somme numérique révélatrice de l'incidence d'unités de temps post - enfouissement des résidus culture cellulosique au sol (RCsol) plus, ou moins, favorables, selon le cas, à un maximum d'activité diazotrophe (Nif) des bactéries du sol

**dose X** (dX) : dose X (kg-N, ou "unités d'azote" / ha) - son calcul peut être aussi simple qu'un [(coefficient d'utilisation de l'N spécifique à une variété de la culture x l'objectif de rendement) - les reliquats d'azote automnaux sur 60 cm], mais aussi très complexe et exhaustif comprenant une foule *d'écritures opérationnelles* et un certain *bilan prévisionnel d'azote* (Comifer 2013), la plupart de ces écritures devant in fine être de toutes façons grossièrement estimées à l'aide d'abaques plus ou moins bien renseignés.

**RCsol** : résidus de culture cellulosiques au sol - Ceux-ci sont avantageusement pailleux, y compris de maïs - grain, bien que les RCsol de cultures de tournesol et de colza sont s'avèrent aussi de bons substrats pour l'AZB.

**Stics** : Simulateur mulTIdisciplinaire pour les Cultures Standard - Brisson et al. 2008 / http://www6.paca.inra.fr/stics

**EPIC** : Erosion productivity impact calculator - Williams 1990, Williams et al. 1995, 2000 / http://epicapex.tamu.edu/epic/

**AGNPS** : Agricultural non-point source pollution model - Bingner et Theurer 2009, Bingner et al. 2001 / http://go.usa.gov/KFO

**WXGN3020** : générateur de données météorologiques à partir de <u>données climatologiques</u> mensuelles utilisables par les modélisations dynamiques de types Stics, EPIC, AGNPS ... (Williams 1990, Williams et al. 1995, 2000) - http://epicapex.tamu.edu/downloads/model-executables/wxg n-v-3020/

**MODWEC** : générateur (Liu et al. 2009) - de données météorologiques à partir de <u>données climatologiques complémentaires</u> de WXGN3020 capable de tenir compte du lien thermique entre journées pluvieuses et température ambiantes minimax - http://www.eawag.ch/forschung/siam/software/modawec/index

**Nm** : azote minéral du sol (kg-N/ha, mg-N/kg-sol) - il s'agit généralement de la somme des espèces nitrique (NO3-) et ammoniacale (NH4+)

**Nif** (NIF, nif) : alias nitrogenase, le complexe enzymatique impliqué dans la réduction de l'azote diatomique ; ici Nif fait référence à l'activité diazotrophique des bactéries du sol

**FRANPIS** : fertilisation raisonnée des apports d'engrais azotés avec pilotage intra - saisonnier - Cette approche est aujourd'hui déclinée sous plusieurs appellations, eg. Jubil™, N-Testeur™, etc.

**inter-culture** : période entre la récolte d'une culture agronomique et le *démarrage* de la culture suivante. Dans le cas d'une culture de céréalière d'hiver comme le blé, il s'agit de la période entre le semis automnal, après enfouissement des résidus de culture au sol, et la sortie d'hiver avant l'apport de la première fraction, N1, de la dX.

**jpe** : jours post enfouissement (des résidus de culture (cellulosique) de la culture précédente - Ces jpe vont servir de base au calcul de l'incidence d'unités de temps post - enfouissement des résidus culture cellulosique au sol (RCsol) plus, ou moins, favorables, selon le cas, à un maximum d'activité diazotrophe (Nif) des bactéries du sol

**Vadose** (zone), ou *zone non saturée* : partie du sol et/ou du sous-sol située à l'interface entre atmosphère-pédosphère et la nappe phréatique. Dans cette zone, les pores du sol sont partiellement remplis d'eau (à l'exception de la frange capillaire) et de gaz (le plus souvent de l'air), contrairement à la *zone saturée en eau* (ou aquifères), dans laquelle la totalité du système poreux est rempli d'eau (source : Wikipédia).

## DIVULGATION DE L'INVENTION

*Problème technique*

Le problème technique peut se résumer, faute de véritables protocoles FRANPIS facilement mise en œuvre par l'utilisateurs, à savoir comment préconiser un rationnement non contre-productif de la dX, notamment en présence d'AZB des RCsol. Cette dX demeure néanmoins pré - calculée conventionnellement (eg. Comifer 2013), mais son susdit rationnement devra se faire sans porter atteinte au potentiel de rendement, la teneur en protéine et/ou la qualité des grains, ou plus généralement de la récolte ; ce rationnement de la dX ne devra plus nuire à l'efficacité relative d'une éventuelle AZB des RCsol.

*Solution technique*

A titre de solution technique je propose au lieu de rationner la dose X d'engrais, de recalculer celle-ci en intégrant, non pas la fourniture du sol en Nm via la minéralisation de l'humus, mais plutôt en fonction de la réserve intra - saisonnière d'Nm fonction elle de l'immobilisation d'Nm pendant l'inter - culture, mais aussi et surtout d'une certaine réserve d'N d'origine diazotrophe. Cette dX sera ainsi établie peu avant l'apport d'N1, <u>la première fraction</u> de la dX apportée dès la sortie d'hiver. Ainsi, le rationnement de la dX, s'il y a, rationnement recherché (escompté) par les utilisateurs d'AZB des RCsol, ce fera donc en pleine connaissance de cause, et sur la base d'un certain "gonflement" de cette réserve hivernale d'azote d'origine diazotrophe.

A priori, évaluer cette contribution d'AZB nécessiterait *deux* échantillons des teneurs en azote minéral (Nm) de sols, un à l'automne, et l'autre en sortie d'hiver. Pourtant, il s'avère que cette contribution diazotrophique est dépendant des conditions agro - climatiques, et pourra donc être avantageusement modélisée à partir de telles données (Jego *et al.* 2012) ; ces estimés pourraient ainsi remplacer un deuxième prélèvement de sol en sortie d'hiver, ce qui, en plus, rendra ce nouveau calcul de la dose X d'autant plus simple. Au lieu, et plus simplement, il est proposé ici de déterminer à l'automne, et qu'à l'automne, les teneurs en Nm du sol, au lieu de la plus usuelle détermination des teneurs des sols en Nm en "sortie d'hiver" comme le propose le Comifer 2013. Cette détermination automnale d'Nm a elle l'avantage d'être logistiquement plus simple, i.e. les déterminations Nm sortie hiver se font souvent dans la hâte, encombrant ainsi d'autant plus les chaînes d'analyses des laboratoires commerciaux déjà surchargées à cette époque de l'année. Les préconisations de rationnement, ou non rationnement, des dX pré - calculées selon le Comifer (2013) se feront ainsi plus sereinement.

Il ne s'agit donc plus d'assurer FRANPIS lors de l'azotobactérisation des résidus de culture cellulosiques au sol, mais bien d'ajuster dX en fonction d'une certaine contribution du potentiel diazotrophe du sol pendant l'interculture. Cela évite d'avoir à calculer la dose X sur la base d'hypothétiques fournitures en N par voie de minéralisation de la matière organique du sol. AZB ayant aussi participée à l'immobilisation d'Nm, la dX ainsi calculée ne peut être qu'adaptée à cette nouvelle donne. Du coup, le rationnement de dX en présence d'AZB ne sera plus fait à l'aveuglette, ce qui ne pourra que contribuer à l'efficacité de cette nouvelle biofertilisation.

Concrètement, il s'agit donc d'une méthode pour le diagnostic de l'état diazotrophe de sols arables et de préconisation de la fertilisation de la culture en cours comportant ;

> seulement le relevé automnal de la teneur en azote minérale (Nm) des sols en surface par échantillonnage du sol, donc sans avoir à prélever pour analyse l'ensemble de la zone d'enracinement et/ou de la zone vadose ;

> un indice intégral, dit ici à titre d'exemple indice diazoto - molaire (IDM), constitué d'une somme numérique révélatrice de l'incidence d'unités de temps post - enfouissement des résidus culture cellulosique au sol (RCsol) plus, ou moins, favorables, selon le cas, à un maximum d'activité diazotrophe (Nif) des bactéries du sol ;

> une préconisation concernant le rationnement de la dose X (dX) préalablement calculée. et donc quatre étapes pour le calcul de cet indice, à savoir ;

(i) la détermination de la météorologie affectant la cinétique des concentration de l'azote minéral (Nm) dans la tranche supérieure de la zone vadose du sol par génération de la pluviométrie à partir de moyennes climatiques mensuelles,

(ii) le suivi de la milli - molarité (mM) en Nm de la solution du sol à travers le temps, avantageusement suivant l'enfouissement des résidus de culture cellulosiques au sol (RCsol), en fonction du nombre de jours post-enfouissement (jpe),

(iii) la quantification du degré de rétroaction négative par Nm de la diazotrophie (Nif) de la flore bactérienne du sol par rapport à son activité maximale fonction de la susdite mM d'Nm de la solution du sol, et

(iv) l'intégration à travers le temps (jpe), avantageusement et à titre d'exemple par sommation, d'incréments journaliers (jpe) de la fraction, avantageusement exprimée en pourcentage, de l'activité diazotrophique (Nif) de la flore bactérienne du sol par rapport à son maximum lorsque la milli - molarité de l'azote minéral de la solution du sol, mM-Nm, $\approx 0,00$ (i.e. $\%NIF_{max}$).

La détermination de la météorologie affectant la cinétique de l'azote minéral (Nm) dans la tranche supérieure de la zone vadose du sol par génération de la pluviométrie à partir de moyennes climatiques mensuelles peut être effectuée comme suit ;

> prélever un échantillon de terre avant le semis la culture d'hiver ; avantageusement cette parcelle reçoit les RCsol de la culture précédente, l'IDM étant particulièrement utile en ce sens, surtout si ces RCsol sont (azoto)bactérisés et enfouis au sens de Claude et Fillion 1994 par exemple ;

> analyser pour sa teneur en azote minéral (Nm) ledit échantillon ; ces analyses sont routinière chez la plupart des laboratoires d'analyses agricole (eg. www.sadef.fr), et doivent ici inclure les valeurs pour la teneur en eau (eg. %, ou encore mm), la profondeur (cm) de la couche arable échantillonnée, ainsi que sa densité apparente (eg. g/cm$^3$) ;

> relever mensuellement la climatologique pour la station météorologique la plus rapprochée ; ces relevés vont comporter les températures minimax moyennes mensuelle, les cumuls mensuels de la pluviométrie, et avantageusement le nombre de jours pluvieux pour chacun des mois ;

> générer la météorologie journalière ; pour ce faire il existe bon nombre de "générateurs stochastiques", eg. WXGN320 (Williams 1990, Williams et al. 1995, 2000), ou encore en combinaison MODAWEC (Liu et al. 2008, 2009), qui peuvent en ce sens "convertir" ces valeurs climatiques moyennes mensuelles en météorologie journalière ;

La détermination de la cinétique de l'azote minéral (Nm) dans la tranche supérieure de la zone vadose du sol par génération de la pluviométrie à partir de moyennes climatiques mensuelles est effectuée que pour la surface du sol arable sur un profondeur de 0 à 50, plus particulièrement de 0 à 40 cm, et avantageusement de 0 à 30 cm.

Le suivi de la milli - molarité (mM) en Nm de la solution du sol à travers le temps, avantageusement suivant l'enfouissement des résidus de culture cellulosiques au sol (RCsol), en fonction du nombre de jours post-enfouissement (jpe) peut être effectué comme suit ;

> calculer journalièrement la concentration de la solution du sol à l'aide de la susdite météorologie et analyses de l'azote minéral de la terre automnale ; pour ce faire il existe des modèles des processus sol/plant informatisés et assez élaborés (eg. Stics - Brisson et al. 2008 / http://www6.paca.inra.fr/stics, EPIC - Williams 1990, Williams et al. 1995, 2000 / http://epicapex.tamu.edu/epic/, ou encore AGNPS - Bingner et Theurer 2009, Bingner et al. 2001 / http://go.usa.gov/KFO). Cela dit, l'utilisation commercial de ces logiciels étant restreinte, il faudra développer à partir de ces applications des "méta - modèles" résumant à l'aide d'équations multi - variables comportant les susdites variables (i.e. teneurs en Nm des sol, cumuls pluviométriques, teneurs en argile du sol, etc.) ;

La quantification du degré de rétroaction négative par Nm de la diazotrophie (Nif) de la flore bactérienne du sol par rapport à son activité maximale fonction de la susdite mM d'Nm de la solution du sol peut, elle, être effectuée comme suite ;

➢ appliquer cette concentration, cette milli - molarité, en azote minéral de la solution du sol à une équation empirique décrivant l'activité décroissante de la diazotrophie en réponse à une augmentation de la milli - molarité ambiante de l'azote minéral ; on obtient ainsi un pourcentage de l'activité diazotrophique, Nif, maximale lorsque ladite milli - molarité en Nm de la solution du sol (mM-Nm) est nulle ;

Enfin, l'intégration à travers le temps (jpe), avantageusement et à titre d'exemple par sommation, d'incréments journaliers (jpe) de la fraction, avantageusement exprimée en pourcentage, de l'activité diazotrophique (Nif) de la flore bactérienne du sol par rapport à son maximum lorsque la milli - molarité de l'azote minéral de la solution du sol, mM-Nm, $\approx 0,00$ (i.e. %NIF$_{max}$) peut être effectuée comme suit ;

➢ faire la somme sur une certaine période, dite inter - culture, de ces pourcentages, %NIF$_{max}$, après l'enfouissement automnale des résidus de culture cellulosiques, avantageusement (azoto)bactérisés ; cette somme, généralement d'octobre à fin février / début mars (eg. ici sur 144 jours post-enfouissement (jpe)) est dite indices diazoto - molaire ou IDM.

In fine, il s'agit donc d'une méthode pour le diagnostic de l'état diazotrophe de sols arables et de préconisation de la fertilisation de la culture en cours selon une quelconque des revendications précédentes caractérisée en ce que la préconisation concernant le rationnement de la dose X (dX) préalablement calculée est effectuer à partir du diagnostic de l'état diazotrophique du sol, en ce sens que les IDM les plus élevés sont révélateurs d'une activité Nif des populations bactériennes du sol somme tout assez importante à travers l'inter - culture capable d'assurer une réserve d'azote d'origine diazotrophique, cette réserve pouvant ainsi re-larguer d'appréciables quantités de d'azote minéralisable en substituts d'un certain nombre d'unités d'azote (kg-N d'engrais N / ha) autrement amenés au sol via dX, la dX pouvant ainsi être rationnée à hauteur de, généralement et à titre d'exemple, une vingtaine d'unités (kg-N/ha).

*Avantages apportés et activité inventive*

Lors du calcul et/ou de l'ajustement de la dX, détecter l'incidence de conditions (situations) agro - pédologiques inhibitrices de Nif selon la concentration de la solution du sol en azote minéral, [Nm], est inédit. Cela dit, le dénombrement de telles situations en jours normalisés (jn) et sur la base de la solution du sol (i.e. % eau de la macroporosité selon Stics) plutôt que par unité de sol secs est inattendu, de tels [Nm] ayant toujours été rapportées in fine en termes de kg-Nm/ha ou mg-Nm/kg-sol à titre de "reliquats" d'Nm, la concentration et la proportion de la solution du sol par rapport à la quantité de sol sec ayant été considérée comme trop variable dans le temps et du coup trop peu commode pour exprimer de tels reliquats / [Nm]. Pourtant, c'est bel et bien la [Nm] de la solution du sol, et non plus simplement [Nm] par unité pondérale de sol, frais ou sec, que "vivent" les AZB, l'activité de ces dernières étant ici responsable de l'éventuelle ajustement à la baisse (minoration) de la dose X (dX).

## BRÈVE DESCRIPTION DES DESSINS ET FIGURES

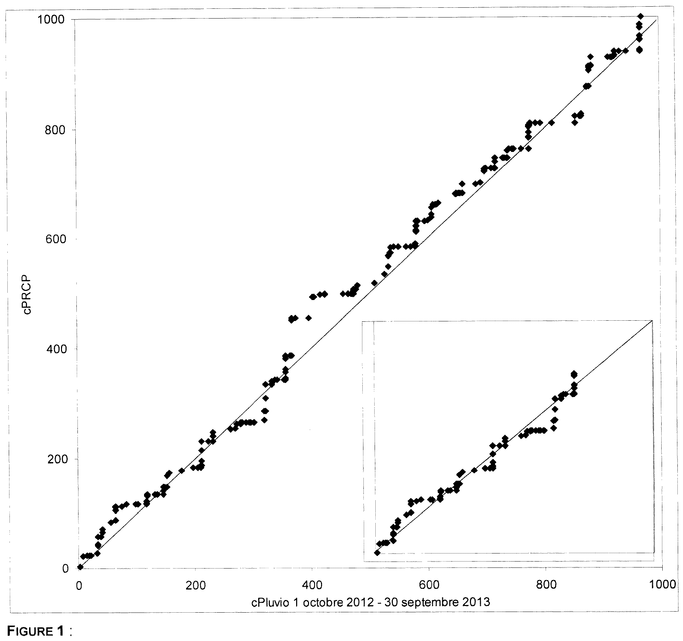

**Figure 1** : Première étape dans le calcul d'IDM ; la modélisation de la cinétique de l'azote minéral dans la zone vadose du sol - génération de la pluviométrie à partir de moyennes climatiques mensuelles. Ici il s'agit de la période allant du 1ier octobre 2012 et au 30 septembre 2013 pour un site en Alsace (68700 Aspach le Bas). Les générateurs météorologiques WXGN3020 (http://epicapex.tamu.edu/downloads/model-executables/wxgn-v-3020/) et MODWEC (Liu et al. 2009 ; http://www.eawag.ch/forschung/siam/software/modawec/index) génère l'incidence et la quantité d'évènements pluvieux à partir d'une série de statistiques mensuelles ; les moyenne des températures aériennes journalière minimales et maximales au sol, leurs écarts-types, le cumul de la pluviométrie pour le mois, ainsi que le nombre de jours pluvieux dans le mois. Il est aussi possible de préciser la probabilité de transition W/W et W/D, et un certain indice d'intensité des orages (WI). Ici, les cumuls des données journalières observées (cPluvio) du 1ier octobre 2012 au 30 septembre 2013 sont mis en relation avec le cumul du même type d'observation mais simulées (cPRCP) ; en abîmes les graphes pour les périodes inter-culturales du 1ier octobre à fin février de l'année suivante, soit 151 jours. A noter que même s'il n'y a pas de correspondance jour pour jour (nb. il ne s'agit pas ici de météorologie mais bien de climatologie), les cumuls pluviométriques observés et simulés concordent assez bien.

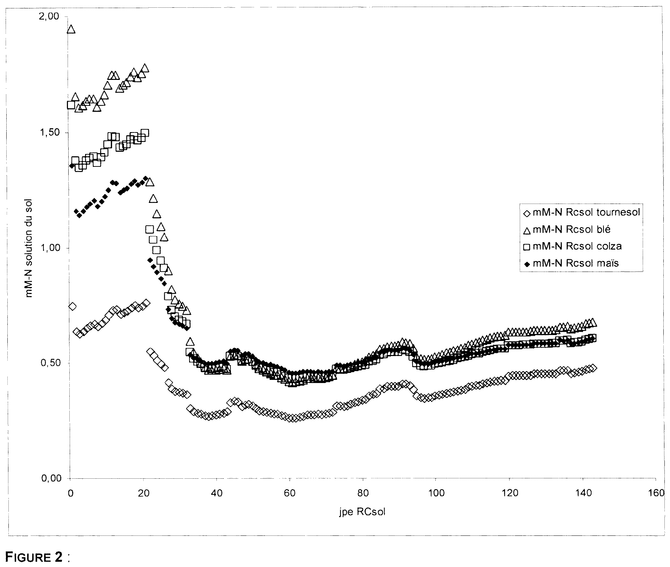

**Figure 2** : : Deuxième étape dans le calcul de l'IDM ; suivi de la (milli)molarité (mM) en N de la solution du sol à travers le temps, avantageusement suivant l'enfouissement des résidus de culture au sol (RCsol), soit par rapport au nombre de jours post-enfouissement (jpe). Cette mM est ici calculée en fonction des teneurs du sol en nitrates ($NO_3^-$) et ammonium ($NH_4^+$), leurs densités apparentes (DA ; $g/cm^3$), la profondeur (Z ; cm) de la couche arable, soit ici de 0 à 30 cm, ainsi que la teneur en eau, $[H_2O]$, de cette couche arable, ou plus précisément selon l'équation suivante ;

$$\frac{([NO_3]/PM_{NO3}/DA_Z) / (Z/10)) + ([NH_4]/PM_{NH4}/DA_Z) / (Z/10))}{[H_2O]/100}$$

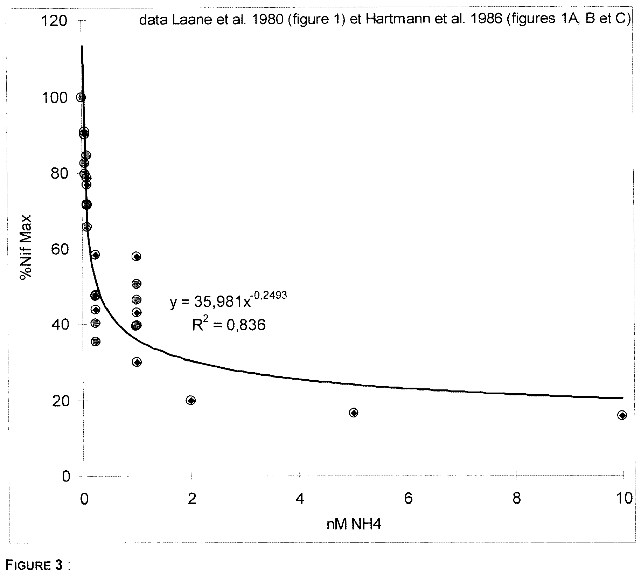

**Figure 3** : Troisième étape dans le calcul d'IDM ; quantifier le degré de rétroaction négative de la diazotrophie par rapport à son activité maximale. L'activité du complexe enzymatique, la nitrogénase, ou Nif, impliqué dans la diazotrophie bactérienne est contrainte rétroactivement par l'opéron nifLA ce qui entraîne une diminution relativement rapide du taux de réduction de $N_2$, voire approximation de $C_2H_2$. Cette réduction est facilement décrite par une fonction puissance négative de type $a \cdot [mM-N]^{-b}$ comprenant ici des données d'études publiées (Laane et al. 1980, Hartmann et al. 1986) ; ce paramétrage sera peaufiné à l'aide de nouvelle données dédiées au fils tu temps.

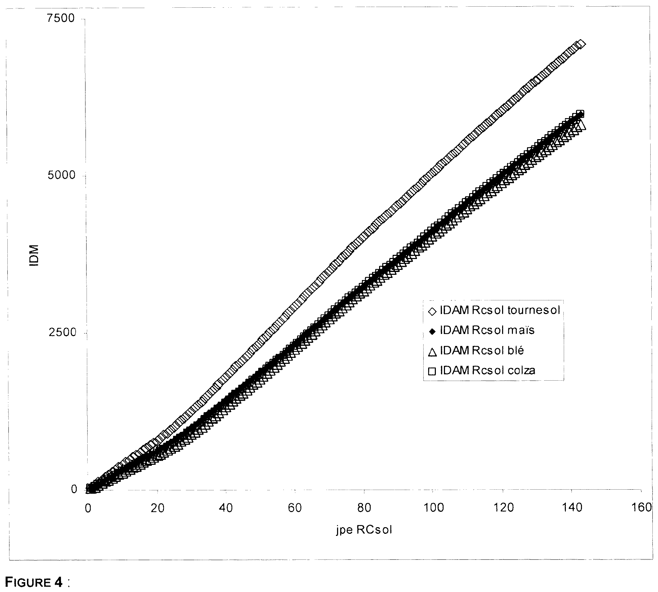

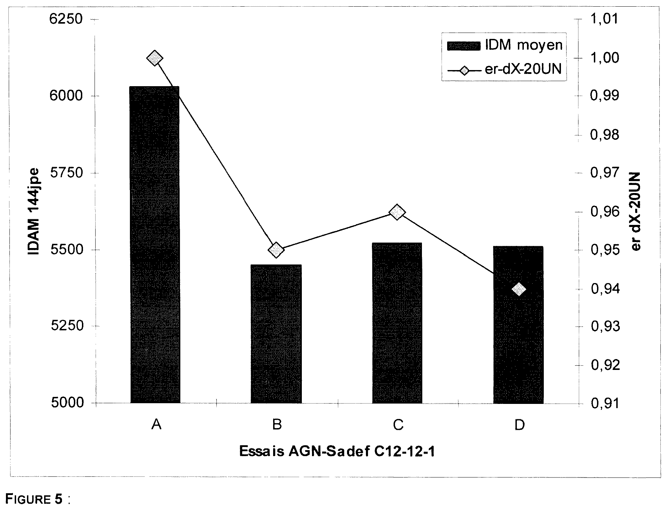

**Figure 4** : Quatrième étape dans le calcul de l'IDM ; intégrer par incréments journaliers le pourcentage de NIF par rapport à son maximum lorsque mM-N ≈ 0,00. Sur une période de l'ordre de 5 à 6 mois, soit à titre d'exemple 144 jours post-enfouissement (jpe) des différents résidus de culture (cellulosiques) au sol (RCsol) en octobre 2012 (cf. Figure 1). L'IDM est donc la somme, le cumul, de ces pourcentages par rapport aux Nif maxima (%Nif Max). On notera que %Nif Max de doit pas en principe dépasser 100%, et que la valeur ultime d'IDM dépendra elle de la période d'intégration (de cumul) ; un IDM entre 5 000 et 10 000 est en ce sens le plus usuel. In fine, plus l'IDM sera important, plus la "bulle" diazotrophe, en sorte, sera importante, et plus le re-largage de l'azote d'origine diazotrophe au système sol/plante sera important ; le cas échéant, il est raisonnable de croire que la dose X (dX) calculée pourra être rationnée à hauteur de cette apport d'N d'origine diazotrophe (cf. Figure 5).

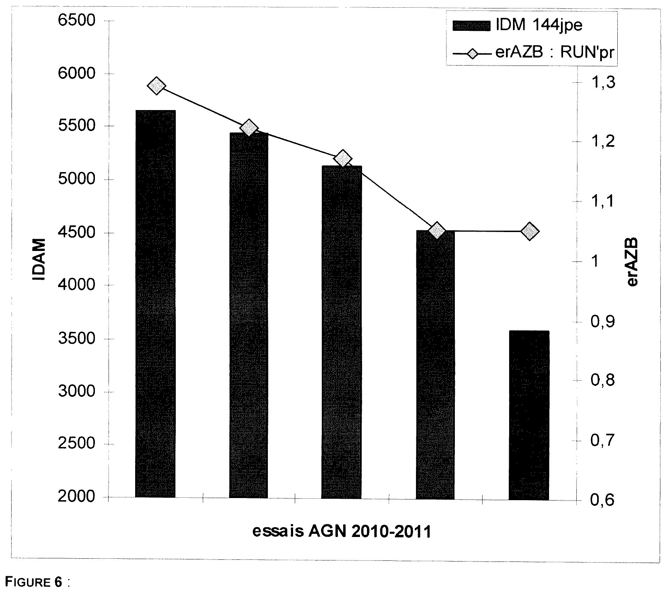

**Figure 5** : Une première validation de l'application de l'IDM lors du rationnement de la dX ; les parcelles "subissant", en sorte, un rationnement de la dose dX à hauteur de 20UN (Tableau 1) vont pouvoir maintenir leur rendement en blé tendre d'hiver (BTH) que si l'IDM est élevé. Dans le cas contraire, l'efficacité relative (er) de cette dX - 20UN sera négative, ou plus exactement ici infra 1,00. En ce sens, seules les parcelles de BTH de l'essai A purent subir un rationnement de la dX sans préjudice. En principe, de ce rationnement de la dX sera surtout préconisé lors d'azotobactérisation des RCsol, cette pratique devant, aux yeux de l'utilisateur, engendrer des économies d'azote avantageusement escomptables dès la sortie d'hiver (cf. Figure 6). (Nb. Essais A, B, C et D comportent des résidus de culture au sol enfouis de tournesol, blé, colza et maïs-grain, respectivement.)

**Figure 6** : Une deuxième validation de l'IDM lors de l'azotobactérisation (AZB) des RCsol ; L'AZB des RCsol implique souvent un rationnement de la dX. En principe cette pratique n'est pas la plus recommandable, mais demeure néanmoins populaire auprès des utilisateurs. L'IDM étant calculé *avant* l'apport de la première fraction de la dX, il est possible de savoir à ce moment là s'il est judicieux de rationner cette dX à hauteur de quelques 20UN, voire plus. En ce sens, cinq (5) parcelles agriculteurs en France furent ainsi azotobactérisés (Tableau 2), le plus souvent avec une dX ainsi réduite ; l'efficacité relative de ces AZB fut elle aussi a priori appréciable à l'aide d'IDM.

## MODE DE RÉALISATION PRÉFÉRÉ DE L'INVENTION

Sur chaque parcelle qui doit être ainsi diagnostiquée, il faut donc ;

a) prélever un échantillon de terre avant le semis de la culture d'hiver ; avantageusement cette parcelle reçoit les RCsol de la culture précédente, l'IDM étant particulièrement utile en ce sens, surtout si ces RCsol sont (azoto)bactérisés et enfouis au sens de Claude et Fillion 1994 par exemple ;

b) analyser pour sa teneur en azote minéral (Nm) ledit échantillon ; ces analyses sont routinières chez la plupart des laboratoires d'analyses agricole (eg. www.sadef.fr), et doivent ici inclure les valeurs pour la teneur en eau (eg. %, ou encore mm), la profondeur (cm) de la couche arable échantillonnée, ainsi que sa densité apparente (eg. $g/cm^3$) ;

c) obtenir les relevés mensuels climatologiques de la station météorologique la plus rapprochée ; ces relevés vont comporter les températures minimax moyennes mensuelle, les cumuls mensuels de la pluviométrie, et avantageusement le nombre de jours pluvieux pour chacun des mois ;

d) générer la météorologie journalière en utilisant des générateurs stochastiques, eg. WXGN320 (Williams 1990, Williams et al. 1995, 2000), ou encore en combinaison MODAWEC (Liu et al. 2008, 2009) capables de convertir ces valeurs climatiques moyennes mensuelles en météorologie journalière ;

e) calculer par incréments journaliers la concentration en Nm de la solution du sol à l'aide de la susdite météorologie et analyses de l'azote minéral de la terre automnale à l'aide de modèles des processus sol/plant tels que Stics - Brisson et al. 2008 / http://www6.paca.inra.fr/stics, EPIC - Williams 1990, Williams et al. 1995, 2000 / http://epicapex.tamu.edu/epic/, ou encore AGNPS - Bingner et Theurer 2009, Bingner et al. 2001 / http://go.usa.gov/KFO, ou encore d'éventuels *méta - modèles* comportant des équations multi - variables comportant les plus déterminantes des susdites variables pédoclimatiques ;

f) appliquer cette concentration (mM) en azote minéral (Nm) de la solution du sol à une équation empirique décrivant l'activité décroissante de la diazotrophie en réponse à une augmentation de la milli - molarité ambiante de la l'azote minéral ; on obtient ainsi un pourcentage de l'activité diazotrophique, Nif, maximale lorsque ladite milli - molarité est nulle ;

g) faire la somme sur une certaine période, dite *inter- culture*, de ces pourcentages, %NIF$_{max}$, après l'enfouissement automnale des RCsol, avantageusement (azoto)bactérisés ; cette somme, généralement d'octobre à fin février / début mars (eg. ici sur 144 jours post-enfouissement (jpe)) est dite indices diazoto - molaire ou IDM.

h) effectuer le diagnostic → préconisation, les IDM les plus élevés étant révélateurs d'une activité Nif somme tout assez importante à travers l'inter-culture ; le cas échéant, la réserve d'azote d'origine diazotrophique est importante, cette "bulle" pouvant relarguer d'appréciables quantités de d'azote *minéralisable* pouvant se substituer à un certain nombre d'unités d'azote normalement (autrement) amener au sol par la dose X ; en d'autre mots, si l'IDM est élevé, la dose X (dX) peut être rationnée, généralement et à titre d'exemple, d'une vingtaine d'unités (kg-N/ha).

## APPLICATIONS BIOINDUSTRIELLES ET AGRONOMIQUES

L'invention est particulièrement applicable au calcul et la préconisation des dX d'N aux grandes cultures d'hiver. Elle est particulièrement utile s'il y a azotobactérisation (AZB) des résidus de culture pailleux (cellulosique) au sol. Pour illustrer ce type d'application agronomique de l'invention, j'ai donc utilisé deux cas de figure ; a) un avec une simple réduction (rationnement) de la dX, et l'autre, b) avec un dX ainsi rationnée en présence d'AZB des RCsol.

a) rationnement de la dX sans contre - performance agronomique

Quatre (4) séries de six (6) parcelles agronomiques de blé tendre d'hiver (BTH) avec différents types de RCsol, A) tournesol, B) blé, C) colza et D) maïs - grain ont été suivies. Il s'agit essentiellement des parcelles appariées l'une à l'autre avec et sans rationnement de la dX à hauteur de 20 UN.
Les IDM pour chacune de ces situations ont été calculés (Tableau 3, Figure 5) à partir des quelques variables fournies par une simple analyse de terre et quelques informations complémentaires (Tableau 1). Les autres variables nécessaires au pilotage de Stics pour ces quatre situations sont celles d'une culture de BTH sur une campagne fournies à titre d'exemple avec l'exécutable Stics (Brisson et al. 2008 ; http://www6.paca.inra.fr/stics). Les données climatologiques alimentant le générateur de météorologie journalière (MODAWEC ; Liu et al. 2008, 2009) afin de constituer les fichiers climatologiques Stics sont celles d'une station météo près d'Aspach-le-Bas (67800, France ; Tableau 2) pour les années 2012 et 2013 ; à titre d'illustration, les cumuls pluviométriques actuels (cPluvio) et générés (cPRCP) pour la période octobre 2012 à septembre 2013 sont comparées graphiquement à la Figure 1.
Comme on peut le voir au Tableau 3 et à la Figure 5, les parcelles "subissant", en sorte, un rationnement de la dose dX à hauteur de 20UN (Tableau 1) vont pouvoir maintenir leur rendement en blé tendre d'hiver (BTH) *que* si l'IDM est relativement élevé. Dans le cas contraire, l'efficacité relative (er) de cette dX - 20UN sera négative, ou plus exactement ici infra 1,00. En ce sens, seules les parcelles de BTH de l'essai A purent subir un rationnement de la dX sans préjudice.

**TABLEAU 3** (Figure 5) : Exemple de parcelles agronomiques avec différents types de résidus de culture cellulosiques au sol (RCsol) précédent le semis d'une culture de BTH avec et sans le rationnement de la dX - on notera que l'efficacité, ou plus exactement la contre - productivité de ce rationnement est nulle (i.e. er dX-20UN $\geq$ 1,0) que pour l'essai avec l'IDM le plus élevé. Nb. L'er est ici établi pour les rendements protéiques, i.e. les rendements en grains x % de protéine des parcelles avec et sans ledit rationnement de la dX de 20 unités d'azote (UN).

| Précédent RCsol | IDM 144 jpe | er dX-20UN |
|---|---|---|
| A. tournesol | 6029 | 1,00 |
| B. BTH | 5452 | 0,95 |
| C. colza | 5524 | 0,96 |
| D. maïs | 5511 | 0,94 |

b) efficacité d'AZB des RCsol avec rationnement de la dX

En principe, de ce rationnement de la dX sera surtout préconisé lors d'azotobactérisation (AZB) des RCsol (eg. Claude et Fillion 2004), cette pratique devant, aux yeux de l'utilisateur, engendrer des économies d'azote avantageusement escomptables dès la sortie d'hiver (cf. Figure 6). (Nb. Essais A, B, C et D comportent des résidus de culture au sol enfouis de tournesol, blé, colza et maïs-grain, respectivement.)

Nous avons donc suivi cinq (5) parcelles agriculteur en termes de d'IDM et d'efficacité relative (er) de l'AZB des différents types de RCsol présent au sol avant le semis conventionnel d'un BTH. Il s'agit cette fois-ci de "bandes" appariées avec et sans l'AZB des RCsol, les bandes azotobactérisées ayant des dX rationnée de 10 à 20 unités d'N (UN ; kg-N/ha) (Tableau 6). Les valeurs pour les quelques variables pédologiques et culturales nécessaire au renseignement des fichiers sols, initialisation et pratiques culturales Stics sont au Tableau 4 ; les données climatologiques pour la génération des valeurs météorologiques journalières sont au Tableau 5.

Il s'agit donc ici de parcelles de BTH dans la région de Toulouse / Agen, la station météo d'où proviennent les données climatologiques sont celles de la station météo de Toulouse - Blagnac (31000, LATD 43:37:24 / LONG 01:22:42). Les procédures de modélisation Stics et MODAWEC sont les mêmes que celles pour le cas de figure précédent.

**TABLEAU 1** : Valeurs variables pour quatre (4) séries de six (6) parcelles de blé tendre d'hiver (BTH), y compris les types de résidus de culture cellulosique au sol (RCsol) et leurs dates (jours juliens ; jj) d'enfouissement à l'automne 2012 (Alsace, France). Comme on peut le voir, les ratios C/N et les densités apparentes (DA ; g/cm3) ne sont pas renseignés et durent être estimés. Selon le cas, ces valeurs sont inscrites dans les fichiers sols, initialisations et pratiques culturales Stics (Brisson et al. 2008 / http://www6.paca.inra.fr/stics) ; les nombreuses autres valeurs paramétriques sont celles fournies à titre d'exemple pour le blé (blé1ans.xml) avec le kit d'installation du logiciel. On notera enfin que seules les teneurs en azote minéral, [Nm], des sols sur 30 cm de profondeur sont nécessaires pour la génération d'IDM l'activité azotobactérienne étant généralement limitée à de telles profondeurs

| Essai 2013 | RCsol | enfouissemnet le jj; | pH | %Argile | Corg g/kg | C/N sol | [Nm] 30cm kg/ha | DA g/cm3 | %H2O |
|---|---|---|---|---|---|---|---|---|---|
| A | tournesol | 297 | 6,6 | 15 | 11,3 | 10 | 19 | 1,35 | 18,6 |
| B | blé | 297 | 5,3 | 18 | 15,7 | 10 | 32,4 | 1,35 | 20,3 |
| C | colza | 297 | 6,6 | 15 | 11,3 | 10 | 39,7 | 1,35 | 20,1 |
| D | maïs-grain | 297 | 7,1 | 12,8 | 10,9 | 10 | 43,8 | 1,35 | 20,4 |

**TABLEAU 2 :** Valeurs mensuelles 2012 et 2013 pour les différentes variables climatologiques permettant d'alimenter WXGN3020 (Williams 1990, Williams et al. 1995, 2000) et MODEWAC (Liu et al. 2008, 2009) de manière à générer les valeurs météorologiques (cf. Figure 1) pour les fichiers climatologiques Stics. Il s'agit ici d'une station météo près d'Aspach-le-Bas (67800, France), lieu approximatif des quatre séries d'essais référencées au Tableau 1. (NB. Pour chaque mois, TMN et TMX - températures C minimax moyennes ; SDMN et SDMN - écartypes mensuelles pour TMN et TMX ; PRCP - cumuls pluviométrique (mm) ; SDRF et SKRF - écartypes et coefficients d'asymétries de la pluviométrie mensuelle ; PW/D et PW/W - probabilités de journées secs et pluvieuses, respectivement, à la suite d'une journées pluvieuse ; DAYP - nombre de jours pluvieux dans le mois.

| année 2012 | janvier | février | mars | avril | mai | juin | juillet | août | septembre | octobre | novembre | décembre |
|---|---|---|---|---|---|---|---|---|---|---|---|---|
| TMX | 4,94 | 0,9 | 14,5 | 13,43 | 20,31 | 22,92 | 23,55 | 26,29 | 19,72 | 14,74 | 9,95 | 5,71 |
| TMN | 0,85 | -5,97 | 1,81 | 4,52 | 9,52 | 12,2 | 13,68 | 14,26 | 9,65 | 7,61 | 3,92 | 0,95 |
| SDMX | 3,09 | 6,89 | 4,28 | 4,72 | 4,56 | 4,71 | 4,33 | 4,61 | 3,91 | 5,58 | 3,64 | 4,6 |
| SDMN | 3,8 | 5,87 | 2,61 | 3,64 | 3,4 | 3,51 | 2,25 | 2,76 | 2,78 | 4,18 | 2,8 | 4,14 |
| PRCP | 130,5 | 8 | 11,5 | 85,5 | 77,8 | 124,00 | 99 | 95 | 59,5 | 87,5 | 157,5 | 196 |
| SDRF | 6,83 | 0,91 | 1,07 | 3,85 | 4,51 | 6,96 | 5,44 | 6,89 | 3,56 | 5,08 | 7,81 | 7,95 |
| SKRF | 1,73 | 3,61 | 3,39 | 1,21 | 2,64 | 2,2 | 2,77 | 2,65 | 1,92 | 2,29 | 1,3 | 1,27 |
| PW\|D | 0,37 | 0,06 | 0,09 | 0,37 | 0,35 | 0,38 | 0,35 | 0,31 | 0,31 | 0,33 | 0,36 | 0,41 |
| PW\|W | 0,62 | 0,31 | 0,34 | 0,62 | 0,6 | 0,63 | 0,6 | 0,56 | 0,56 | 0,58 | 0,61 | 0,66 |
| DAYP | 4,94 | 0,9 | 14,5 | 13,43 | 20,31 | 22,92 | 23,55 | 26,29 | 19,72 | 14;74 | 9,95 | 5,71 |

| année 2013 | janvier | février | mars | avril | mai | juin | juillet | août | septembre | octobre | novembre | décembre |
|---|---|---|---|---|---|---|---|---|---|---|---|---|
| TMX | 3,29 | 2,91 | 6,84 | 14,58 | 16,31 | 23,6 | 28,84 | 26,74 | 20,92 | 17,15 | 7,67 | 7,82 |
| TMN | -0,89 | -2,05 | 0,13 | 5,27 | 8,85 | 12,37 | 16,45 | 14,47 | 12,02 | 9,77 | 3,15 | 0,52 |
| SDMX | 4,18 | 3,52 | 4,66 | 6,23 | 3,69 | 5,2 | 3,75 | 3,91 | 5,02 | 3,96 | 4,32 | 3,21 |
| SDMN | 3,74 | 3,98 | 3,38 | 4,08 | 2,09 | 3,23 | 2,64 | 3,42 | 3,08 | 3,42 | 4,08 | 3,38 |
| PRCP | 62,5 | 91 | 55 | 97 | 83,5 | 43 | 43,5 | 54 | 98 | 138,5 | 146,5 | 96,5 |
| SDRF | 4,59 | 7,09 | 2,58 | 6,38 | 3,85 | 3,21 | 3,28 | 3,82 | 7,29 | 4,3 | 6,88 | 7,15 |
| SKRF | 3,13 | 3,68 | 1,96 | 1,91 | 2,02 | 3,09 | 2,3 | 2,32 | 2,77 | 0,45 | 1,31 | 3,2 |
| PW\|D | 0,3 | 0,35 | 0,33 | 0,3 | 0,41 | 0,24 | 0,22 | 0,25 | 0,34 | 0,41 | 0,37 | 0,32 |
| PW\|W | 0,55 | 0,6 | 0,58 | 0,55 | 0,66 | 0,49 | 0,47 | 0,5 | - 0,59 | 0,66 | 0,62 | 0,57 |
| DAYP | 3,29 | 2,91 | 6,84 | 14,58 | 16,31 | 23,6 | 28,84 | 26,74 | 20,92 | 17,15 | 7,67 | 7,82 |

Comme on peut le voir au Tableau 6 et à la Figure 6, les efficacité relative (er) d'AZB en présence de tels rationnement de la dX les plus importantes sont grosso modo associées au IDM les plus élevés ; ici, des IDM de moins de 5000 semblent indiquer que l'AZB des RCsol aurait profitées d'une pleine dX, sans rationnement.

**TABLEAU 6** (Figure 6) : Exemple de parcelles agronomiques de BTH avec et sans azotobactérisation (AZB) des résidus de culture cellulosiques au sol, avec et sans rationnement de la dose X (dX) ; on notera qu'étant donné un rationnement de la dX, les IDM les plus élevés sont associés aux efficacités relatives d'AZB (er ; ratios avec/sans AZB) des RCsol les plus importantes. Nb. L'er d'AZB est ici établi pour les rendements unitaires protéiques, i.e. le nombre de kg de protéine de blé récolté par unité d'azote kg-N/ha fertilisant apportée.

| No. Parcelle 2011 | IDM 144 jpe | erAZB | dUN | Précédent RCsol | rNm |
|---|---|---|---|---|---|
| 7 | 5647 | 1,29 | -20 | sorgho | 9,9 |
| 15 | 5446 | 1,22 | -20 | BDH | 8 |
| 19 | 5141 | 1,17 | -10 | maïs | 21 |
| 6 | 4530 | 1,05 | -20 | maïs | 25 |
| 14 | 3600 | 1,05 | -20 | BDH | 53 |

*Références, bibliographie et brevets pertinents*

BINGNER, R. L. AND F.D. THEURER. 2009. AGNPS WEB SITE. INTERNET AT HTTP://WWW.ARS.USDA.GOV/RESEARCH/DOCS.HTM?DOCID=5199

BINGNER, R. L. AND F. D. THEURER. AGNPS 98: A SUITE OF WATER QUALITY MODELS FOR WATERSHED USE. IN PROCEEDINGS OF THE SEDIMENT: MONITORING, MODELING, AND MANAGING, 7TH FEDERAL INTERAGENCY SEDIMENTATION CONFERENCE, RENO, NV, 25-29 MARCH 2001: SUBCOMMITTEE ON SEDIMENTATION OF THE INTERAGENCY ADVISORY COMMITTEE ON WATER DATA, P. VII-1 - VII-8. 2001.

BRISSON, N, (EDITEUR) ; LAUNAY, M, (EDITEUR) ; MARY, B, (EDITEUR) ; BEAUDOIN, N, (EDITEUR), CONCEPTUAL BASIS, FORMALISATIONS AND PARAMETERIZATION OF THE STICS CROP MODEL. VERSAILLES (FRA) : EDITIONS QUAE ; 2008. 304 P.

BRISSON N., RUGET F., GATE P., LORGEOU J., NICOULLAUD B., TAYOT X., PLENET D., JEUFFROY M.H., BOUTHIER A., RIPOCHE D., MARY B., JUSTES E. 2002A. STICS: A GENERIC MODEL FOR THE SIMULATION OF CROPS AND THEIR WATER AND NITROGEN BALANCES. II. MODEL VALIDATION FOR WHEAT AND CORN. AGRONOMIE, 22, 69-93.

CLAUDE, PP ET L FILLION. 2004. EFFET DE L'APPORT D'UN INOCULUM BACTÉRIEN AUX RÉSIDUS DE CULTURE DE MAÏS-GRAIN AU SOL SUR LE RENDEMENT ET LA QUALITÉ DE BLÉS D'HIVER PANIFIABLES EN FRANCE. AGROSOLUTIONS JUIN 2004, VOL. 15, NO 1 PAGES 23-29

COMIFER 2013. CALCUL DE LA FERTILISATION AZOTÉE : GUIDE MÉTHODOLOGIQUE POUR L'ÉTABLISSEMENT DES PRESCRIPTIONS LOCALES - CULTURES ANNUELLES ET PRAIRIES. BROCHURE ÉDITÉE PAR LE COMIFER / LE DIAMANT A, 92909 PARIS LA DÉFENSE CEDEX, TÉL. : 01 46 53 10 75 / DÉPÔT LÉGAL : AVRIL 2011, ISBN 978-2-910393-09-0

DE WIT C.T. 1978. SIMULATION OF ASSIMILATION RESPIRATION AND TRANSPIRATION OF CROPS. IN SIMULATION MONOGRAPHS. PUDOC, WAGENINGEN, THE NETHERLANDS. PP. 141.

HARTMANN, A, H FU ET RH BURRIS. 1986. REGULATION OF NITROGENASE ACTIVITY BY AMMONIUM CHLORIDE IN AZOSPIRILLUM SPP. JOURNAL OF BACTERIOLOGY, MAR. 1986, P.864-870

JEGO G.. SANCHEZ-PEREZ J.M., JUSTES E., 2012 PREDICTING SOIL WATER AND MINERAL NITROGEN CONTENTS WITH THE STICS MODEL FOR ESTIMATING NITRATE LEACHING UNDER AGRICULTURAL FIELDS, AGRICULTURAL WATER MANAGEMENT 107 54-65 LAANE, C, W KRONE,W KONINGS,H HAAKER ET CVEEGER. 1980.SHORT TERM EFFECT OF AMMONIUM CHLORIDE ON NITROGEN FIXATION BY AZOTOBUCTER VINELANDII AND BY BACTEROIDS OF RHIZOBIUM LEGUMINOSUVUM. EUR. J. BIOCHEM. 103, 39-46 (1980)

LIU J., WILLIAMS J.R., WANG X., YANG H., 2009. USING MODAWEC TO GENERATE DAILY WEATHER DATA FOR THE EPIC MODEL. ENVIRONMENTAL MODELLING & SOFTWARE 24 (5): 655-664.

LIU J., FRITZ S., VAN WESENBEECK C.F.A., FUCHS M., OBERSTEINER M., YANG H., 2008. A SPATIAL EXPLICIT ASSESSMENT OF CURRENT AND FUTURE HOTSPOTS OF HUNGER IN SUB-SAHARAN AFRICA IN THE CONTEXT OF GLOBAL CHANGE. GLOBAL AND PLANETARY CHANGE.64 (3-4): 222-235.

THEURER, F.D., R.L. BINGNER, W. FONTENOT, AND S.R. KOLIAN. 1999. PARTNERSHIPS IN DEVELOPING AND IMPLEMENTING AGNPS 98: A SUITE OF WATER QUALITY MODELS FOR WATERSHED USE. IN PROCEEDINGS OF THE SIXTH NATIONAL WATERSHED CONFERENCE, 16-19 MAY 1999, AUSTIN, TEXAS. 10 PG.

WHISLER J.R., ACOCK B., BAKER D.N., FYE R.E., HODGES H.F., LAMBERT J.R., LEMMON H.E., MCKINION J.M., REDDY, V.R. 1986. CROP SIMULATION MODELS IN AGRONOMIE SYSTEMS, ADV. AGRON. 40, 141-208.

WILLIAMS JR. (1990) THE EROSION PRODUCTIVITY IMPACT CALCULATOR (EPIC) MODEL: A CASE HISTORY. PHIL. TRANS. R. SOC. LOND. 329:421-428.

WILLIAMS JR. (1995) THE EPIC MODEL. PP. 909-1000 IN COMPUTER MODELS OF WATERSHED HYDROLOGY (ED, SINGH VP). WATER RESOURCES PUBLICATIONS, HIGHLANDS RANCH, CO.

WILLIAMS JR, ARNOLD JG & SRINIVASAN R. (2000) THE APEX MODEL. BRC REPORT NO. 00-06. TEMPLE, TX: TEXAS AGRIC. EXPT. STATION. TEXAS AGRIC. EXTEN. SERVICE. TEXAS A&M UNIV.

**TABLEAU 4 :** Valeurs variables pour cinq (5) parcelles de blé tendre d'hiver (BTH), y compris les types de résidus de culture cellulosique au sol (RCsol) et leurs dates (jours juliens ; jj) d'enfouissement à l'automne 2010 (Midi-Pyrénées , France). Comme on peut le voir, les ratios C/N, les densités apparentes (DA ; g/cm3) et le teneur en C organique des sols ne sont pas renseignés et durent être estimés à partir des classes texturales fournies par l'agriculteur, soit essentiellement argilo-calcaire ou *boulbènes.* Selon le cas, ces valeurs sont inscrites dans les fichiers sols, initialisations et pratiques culturales Stics (Brisson et al. 2008 / http://www6.paca.inra.fr/stics) ; les nombreuses autres valeurs paramétriques sont celles fournies à titre d'exemple pour le blé (blé1ans.xml) avec le kit d'installation du logiciel. On notera enfin que seules les teneurs en azote minéral, [Nm], des sols sur 30 cm de profondeur sont nécessaire pour la génération d'IDM, l'activité azotobactérienne étant généralement limitée à de telles profondeurs.

| Parcelle | Rcsol | enfouissemnet le jj; | pH | %Argile | %Corganqu | C/N sol | [Nm] 30cm | DA g/cm3 | %H2O |
|---|---|---|---|---|---|---|---|---|---|
| 7 | sorgo | 300 | 7 | 33 | 1,5 | 12,5 | 10 | 1,2 | 12 |
| 15 | blé | 308 | 7 | 15 | 1,5 | 12,5 | 8 | 1,2 | 12 |
| 19 | maïs-grain | 274 | 6 | 15 | 1,5 | 10 | 21 | 1,2 | 12 |
| 6 | maïs-grain | 301 | 8,2 | 33 | 1,5 | 12,5 | 25 | 1,2 | 12 |
| 14 | blé | 319 | 6 | 15 | 1,5 | 10 | 53 | 1,2 | 12 |

**TABLEAU 5 :** Valeurs mensuelles 2010 et 2011 pour les différentes variables climatologiques permettant d'alimenter WXGN3020 (Williams 1990, Williams et al. 1995, 2000) et MODEWAC (Liu et al. 2008, 2009) de manière à générer les valeurs météorologiques pour les fichiers climatologiques Stics. Il s'agit ici de la station météo de Toulouse - Blagnac (31000, France), lieu approximatif des cinq parcelles agronomiques référencées au Tableau 4. (NB. Pour chaque mois, TMN et TMX - températures C minimax moyennes ; SDMN et SDMN - écartypes mensuelles pour TMN et TMX ; PRCP - cumuls pluviométrique (mm) ; SDRF et SKRF - écartypes et coefficients d'asymétries de la pluviométrie mensuelle ; PW/D et PW/W - probabilités de journées secs et pluvieuses, respectivement, à la suite d'une journées pluvieuse ; DAYP - nombre de jours pluvieux dans le mois.

| année 2010 | janvier | février | mars | avril | mai | juin | juillet | août | septembre | octobre | novembre | décembre |
|---|---|---|---|---|---|---|---|---|---|---|---|---|
| TMX | 7,57 | 9,99 | 13,31 | 19,59 | 19,89 | 25,37 | 29,77 | 28,58 | 24,83 | 18,33 | 12,66 | 9,21 |
| TMN | 0,97 | 2,05 | 4,28 | 7,68 | 10,49 | 14,6 | 18,05 | 16,43 | 13,12 | 9,87 | 5,71 | 0,82 |
| SDMX | 3,73 | 5,11 | 4,82 | 3,92 | 5,26 | 4,28 | 3,26 | 4,43 | 4,16 | 4,56 | 3,84 | 4,46 |
| SDMN | 4,06 | 4,2 | 5,12 | 3,44 | 3,58 | 2,75 | 2,39 | 2,85 | 3,19 | 4,79 | 4,86 | 5,03 |
| PRCP | 57 | 36,5 | 37,9 | 28,2 | 119,9 | 85,1 | 22,8 | 13,8 | 31,1 | 81,9 | 63,4 | 25,8 |
| SDRF | 3,22 | 2,7 | 2,27 | 2,17 | 8,26 | 5,49 | 2,27 | 1,95 | 2,32 | 10,42 | 3,02 | 3,14 |
| SKRF | 1,93 | 2,86 | 2,37 | 2,37 | 3,33 | 2,08 | 3,49 | 5,46 | 2,95 | 5,18 | 1,97 | 4,95 |
| PW\|D | 0,34 | 0,31 | 0,26 | 0,21 | 0,37 | 0,34 | 0,15 | 0,1 | 0,23 | 0,28 | 0,4 | 0,21 |
| PW\|W | 0,59 | 0,56 | 0,51 | 0,46 | 0,62 | 0,59 | 0,4 | 0,35 | 0,48 | 0,53 | 0,65 | 0,46 |
| DAYP | 17 | 16 | 12 | 8 | 17 | 14 | 4 | 7 | 10 | 10 | 22 | 10 |

| année 2011 | janvier | février | mars | avril | mai | juin | juillet | août | septembre | octobre | novembre | décembre |
|---|---|---|---|---|---|---|---|---|---|---|---|---|
| TMX | 9,25 | 12,35 | 15,03 | 21,84 | 25,5 | 25,45 | 26,65 | 29,85 | 27,94 | **22** | 16,43 | 12,33 |
| TMN | 1,79 | 3,64 | 6,27 | 9,97 | 13,03 | 14,08 | 15,83 | 17,28 | 15,73 | 10,26 | 9,51 | 4,95 |
| SDMX | 4,11 | 3,08 | 3,23 | 3,06 | 3,39 | 4,49 | 3,7 | 3,69 | 3,39 | 4,38 | 4,12 | 2,33 |
| SDMN | 4,36 | 3,48 | 3,72 | 2,82 | 2,33 | 2,64 | 2,04 | 3,05 | 3,31 | 2,93 | 3,82 | 3,59 |
| PRCP | 32,6 | 30 | 36,2 | 37,2 | 20,4 | 38,8 | 73,2 | 36,6 | 30,6 | 21,4 | 23,8 | 41,8 |
| SDRF | 2,59 | 2,26 | 2,39 | 5,5 | 1,93 | 3,35 | 5,72 | 3,57 | 3,27 | 1,63 | 1,79 | 1,92 |
| SKRF | 2,85 | 3,5 | 2,27 | 5,28 | 3,71 | 4,43 | 3,53 | 3,18 | 4,43 | 2,44 | 2,52 | 2,59 |
| PW\|D | 0,24 | 0,28 | 0,26 | 0,2 | 0,15 | 0,28 | 0,3 | 0,21 | 0,22 | 0,17 | 0,18 | 0,37 |
| PW\|W | 0,49 | 0,53 | 0,51 | 0,45 | 0,4 | 0,53 | 0,55 | 0,46 | 0,47 | 0,42 | 0,43 | 0,62 |
| DAYP | 11 | 17 | 12 | 6 | 5 | 13 | 11 | 7 | 8 | 8 | 16 | 26 |

**Revendications**

1. Méthode pour le diagnostic de l'état diazotrophe de sols arables et pour la préconisation de la fertilisation d'une culture en cours par rationnement d'une dose X (dX) d'engrais azoté (N) préalablement calculée comportant ;

➢ un relevé automnal de la teneur en azote minérale (Nm) des sols en surface par échantillonnage, sans avoir à prélever pour analyse l'ensemble de la zone d'enracinement et/ou de la zone vadose, (1) en prélevant un échantillon de terre avant le semis de la culture d'hiver, (2) en analysant cet échantillon pour sa teneur en azote minéral (Nm), et (3) en obtenant les relevés mensuels climatologique comportant les températures minimax mensuelles moyennes, les cumuls mensuels de la pluviométrie,

➢ le calcul d'un indice intégral, dit indice diazoto - molaire (IDM), constitué d'une somme numérique révélatrice de l'incidence d'unités de temps post - enfouissement des résidus de cultures cellulosiques au sol (RCsol) plus, ou moins, favorables, selon le cas, à un maximum d'activité diazotrophe (Nif) des bactéries du sol en faisant (1) la somme sur une certaine période, dite inter - culture, de ces pourcentages, %NIF$_{max}$, après l'enfouissement automnale desdits RCsol, et (2) en effectuant un diagnostique en vue d'une préconisation, les IDM les plus élevés étant ainsi révélateurs d'une activité Nif à travers l'inter-culture produisant une réserve d'azote d'origine diazotrophique pouvant re-larguer d'appréciables quantités de d'azote minéralisable et se substituer à un certain nombre d'unités d'azote normalement (autrement) amener au sol par la dX et

- une préconisation suivant l'IDM déterminé concernant le rationnement d'une dose X (dX) préalablement calculée; et donc aussi les quatre étapes suivantes pour le calcul de ce indice, à savoir ;

(i) la détermination de la météorologie affectant la cinétique des concentrations l'azote minéral (Nm) dans la tranche supérieure de la zone vadose du sol par génération stochastique de la pluviométrie journalière à partir de moyennes climatiques mensuelles, cette tranche supérieure de la zone vadose étant limitée à une profondeur de 0 à 50, plus particulièrement de 0 à 40 cm, et avantageusement de 0 à 30 cm, par modélisation des cinétiques de l'azote minéral comprenant la génération stochastique de la pluviométrie à partir des susdites moyennes climatiques mensuelles générant ainsi l'incidence et la quantité d'évènements pluvieux à partir d'un série de statistiques mensuelles telles que les moyenne des températures aériennes journalière minimales et maximales au sol, leurs écarts-types, le cumul de la pluviométrie pour le mois, ainsi que le nombre de jours pluvieux dans le mois, les probabilités de transition, et un certain indice d'intensité des orages ;

(ii) le suivi de la milli - molarité (mM) en Nm de la solution du sol à travers le temps, avantageusement suivant l'enfouissement des RCsol, en fonction du nombre de jours post-enfouissement (jpe) et calculée en fonction des teneurs du sol en nitrates ($NO_3^-$) et ammonium ($NH_4^+$), sa densité apparente (DA ; $g/cm^3$), la profondeur (Z ; cm) de la couche arable, soit ici de 0 à 30 cm, ainsi que la teneur en eau, [$H_2O$], de cette couche arable, ou plus précisément selon l'équation,

$$\frac{\{[NO_3]/PM_{NO3}/DA_Z\} / (Z/10)\} + \{[NH_4]/PM_{NH4}/DA_Z\} / (Z/10)\}}{[H_2O]/100}$$

et cela encore une fois en (1) générant la météorologie journalière à l'aide de générateurs stochastiques capables de convertir ces valeurs climatiques moyennes mensuelles en météorologie journalière, et (2) en calculant par incréments journaliers la concentration en Nm de la solution du sol à l'aide de la susdite météorologie et analyses de l'azote minéral de la terre automnale à l'aide de modèles dynamiques reconnus des processus sol/plant, ou encore d'éventuels méta - modèles comportant des équations multi - variables comportant les plus déterminantes des susdites variables pédoclimatiques ;

(iii) la quantification du degré de rétroaction négative par l'Nm de la diazotrophie (Nif) de la flore bactérienne du sol par rapport à son activité maximale fonction de la susdite mM d'Nm de la solution du sol, réduction (rétroaction négative) facilement et avantageusement décrite par une fonction puissance négative de type a·[mM-N]$^{-b}$ comprenant des données préalable et/ou publiées quitte à peaufiné ce paramétrage au fils tu temps, en (1) appliquant cette concentration (mM) en azote minéral (Nm) de la solution du sol à une équation empirique décrivant l'activité décroissante de la diazotrophie en réponse à une augmentation de la milli - molarité ambiante de Nm de manière à obtenir ainsi un pourcentage de l'activité diazotrophique, Nif, maximale lorsque ladite milli - molarité est nulle ;

(iv) l'intégration à travers le temps (jpe), avantageusement par sommation, d'incréments journaliers (jpe) de la fraction, avantageusement exprimée en pourcentage, de l'activité diazotrophique (Nif) de la flore bactérienne du sol par rapport à son susdit maximum lorsque la milli - molarité de l'azote minéral de la solution du sol, mM-Nm, $\approx$ 0,00 (i.e. %NIF$_{max}$), et cela sur une période de l'ordre de plusieurs mois comprenant un certain nombre de jours post-enfouissement (jpe) des différents résidus de culture (cellulosiques) au sol (RCsol), l'IDM étant donc ici la somme, le cumul, de ces pourcentage par rapport aux Nif maxima (%Nif Max) ne devant pas en principe dépasser 100%, tandis que la valeur ultime d'IDM dépendra elle de la période d'intégration (de cumul).

2. Méthode pour le diagnostic de l'état diazotrophe de sols arables et pour la préconisation de la fertilisation de la culture selon la revendication précédente **caractérisée en ce que** la détermination de la météorologie affectant la cinétique des l'azote minéral (Nm) dans la tranche supérieure de la zone vadose du sol par génération de la pluviométrie à partir de moyennes climatiques mensuelles peut être effectuée comme suit ;

➢

- prélever un échantillon de terre avant le semis de la culture d'hiver; avantageusement en présence des RCsol de la culture précédente surtout si ces RCsol sont (azoto)bactérisés et enfouis;
- analyser pour sa teneur en azote minéral (Nm) ledit échantillon, y compris des valeurs pour la teneur en eau (eg. % du sol, ou encore mm par hectare), la profondeur (eg. cm) de la couche arable échantillonnée, ainsi que sa densité apparente (eg. g/cm$^3$) ;

➢ relever mensuellement la climatologique de la station météorologique la plus rapprochée, y compris les températures minimax moyennes mensuelles, les cumuls mensuels de la pluviométrie, et avantageusement le nombre de jours pluvieux pour chacun des mois ;

➢ générer la météorologie journalière à partir de la susdite climatologie mensuelle ;

3. Méthode pour le diagnostic de l'état diazotrophe de sols arables et pour la préconisation de la fertilisation de la culture selon la revendication précédente **caractérisée en ce que** la génération de la météorologie journalière se fait à l'aide d'un générateur stochastique éprouvé qui peut en ce sens convertir ces valeurs climatiques moyennes mensuelles en météorologie journalière ;

4. Méthode pour le diagnostic de l'état diazotrophe de sols arables et pour la préconisation de la fertilisation de la culture selon une quelconque des revendications précédentes **caractérisée en ce que** le suivi de la milli - molarité (mM) en Nm de la solution du sol à travers le temps, avantageusement suivant l'enfouissement des résidus de culture cellulosiques au sol (RCsol), en fonction du nombre de jours post-enfouissement (jpe) peut être effectué comme suit ; calculer journalièrement la concentration de la solution du sol à l'aide de la susdite météorologie et analyses de l'azote minéral de la terre automnale.

5. Méthode pour le diagnostic de l'état diazotrophe de sols arables et pour la préconisation de la fertilisation de la culture selon la revendication précédente **caractérisée en ce que** le calcul journalier des concentrations de la solution du sol à l'aide de la susdite météorologie et analyses de l'azote minéral de la terre automnale se fait à l'aide de modèles des processus sol/plant informatisés, ou encore à l'aide de "méta - modèles" développées à l'aide de ces applications et résumant à l'aide d'équations multi - variables comportant les susdites variables (i.e. teneurs en Nm des sol, cumuls pluviométriques, teneurs en argile du sols, etc.).

6. Méthode pour le diagnostic de l'état diazotrophe de sols arables et pour la préconisation de la fertilisation de la culture selon une quelconque des revendications précédentes **caractérisée en ce que** la quantification le degré de rétroaction négative par Nm de la diazotrophie (Nif) de la flore bactérienne du sol par rapport à son activité maximale fonction de la susdite mM d'Nm de la solution du sol peut être effectuée comme suit ; appliquer cette concentration, cette milli - molarité (mM), en azote minéral (Nm) de la solution du sol à une équation empirique décrivant l'activité décroissante de la diazotrophie en réponse à une augmentation de la milli - molarité ambiante de l'azote minéral ; on obtient ainsi un pourcentage de l'activité diazotrophique, Nif, maximale lorsque ladite milli - molarité est nulle ;

7. Méthode pour le diagnostic de l'état diazotrophe de sols arables et pour la préconisation de la fertilisation de la culture selon une quelconque des revendications précédentes **caractérisée en ce que** l'intégration à travers le temps (jpe), avantageusement et à titre d'exemple par sommation, d'incréments journaliers (jpe) de la fraction, avantageusement exprimée en pourcentage, de l'activité diazotrophique (Nif) de la flore bactérienne du sol par rapport à son maximum lorsque la milli - molarité de l'azote minéral de la solution du sol, mM-Nm, $\approx 0{,}00$ (i.e. %NIF$_{max}$) peut être effectuée comme suit ; faire la somme sur une certaine période, dite inter - culture, de ces pourcentages, %NIF$_{max}$, après l'enfouissement automnale des résidus de culture cellulosiques, avantageusement (azoto)bactérisés ;

8. Méthode pour le diagnostic de l'état diazotrophe de sols arables et pour la préconisation de la fertilisation de la culture selon la revendication précédente **caractérisée en ce que** la somme sur une certaine période, dite inter - culture, de ces pourcentages, %NIF$_{max}$, après l'enfouissement automnale des résidus de culture cellulosiques, avantageusement (azoto)bactérisés est dite indice diazoto - molaire ou IDM.

9. Méthode pour le diagnostic de l'état diazotrophe de sols arables et pour la préconisation de la fertilisation de la culture selon les deux revendications précédentes **caractérisée en ce que** la somme sur une certaine période, dite inter - culture, de ces pourcentages, %NIF$_{max}$, après l'enfouissement automnale des résidus de culture cellulosiques, avantageusement (azoto)bactérisés comprend une période post enfouissement des RCsol d'octobre à fin février / début mars, ou encore post l'enfouissement des RCsol à la dite "sortie d'hiver" compté en jours post-enfouissement (jpe).

10. Méthode pour le diagnostic de l'état diazotrophe de sols arables et pour la préconisation de la fertilisation de la culture selon une quelconque des revendications précédentes **caractérisée en ce que** la préconisation concernant le rationnement de la dose X (dX) préalablement calculée est effectuer à partir du diagnostic de l'état diazotrophique du sol, en ce sens que les IDM les plus élevés sont révélateurs d'une activité Nif des populations bactériennes du sol assez importante à travers l'inter-culture capable d'assurer une réserve d'azote d'origine diazotrophique relarguant des quantités de d'azote minéralisable en substituts d'unités d'azote (kg-N d'engrais N / ha) autrement amenés au sol via dX, la dX pouvant ainsi être rationnée à hauteur d'une vingtaine d'unités (kg-N/ha).

**Patentansprüche**

1. Methode zur Diagnose des diazotrophen Zustands von Ackerböden zur Empfehlung der Düngung einer bestehenden Kultur durch Rationierung einer zuvor berechneten Dosis X (dX) Stickstoffdünger (N) bestehend aus:

   ➤ einer herbstlichen Erhebung des Gehalts an mineralischem Stickstoff (Nm) der Böden an der Oberfläche durch Probenahme, ohne dabei die Gesamtheit der Wurzelzone und/oder der ungesättigten Zone beproben zu müssen, (1) durch die Entnahme einer Bodenprobe vor der Aussaat der Winterkultur, (2) durch das Analysieren dieser Probe auf ihren Gehalt an mineralischem Stickstoff (Nm), und (3) durch Sammeln der monatlichen klimatologischen Aufzeichnungen mit durchschnittlichen monatlichen Minimal- und Maximaltemperaturen, die kumulierten monatlichen Niederschlagsmengen,

   ➤ die Berechnung eines integralen Index, dem Diazoto-Molaren Index (IDM), der aus einer numerischen Summe, die das Auftreten von Zeiteinheiten nach dem Einackern von Resten von Cellulosepflanzen in den Boden (RCsol) anzeigt, mehr oder weniger günstig, je nach Fall, für ein Maximum an Diazotropher Aktivität (Nif) der Bodenbakterien, indem man (1) die Summe über einen bestimmten Zeitraum, die sogenannte *zwischen-Kulturen-Periode*, dieser Prozentsätze %NIF$_{max}$, nach dem herbstlichen Vergraben dieser RCsol, macht und (2) eine Diagnose durchführt im Hinblick auf eine Empfehlung, die höchsten IDM sind also die Anzeiger einer Nif-Aktivität während der Zeit der Zwischen-Kultur, die eine Stickstoff-Reserve diazotrophen Ursprungs produziert und damit erheblichen Mengen an *mineralisierbarem* Stickstoff freisetzen kann und so eine bestimmte Anzahl an Einheiten von Stickstoff ersetzen, der normalerweise (andernfalls) durch dX in den Boden gebracht würde, und eine Empfehlung nach dem berechneten IDM betreffend die Bemessung einer zuvor berechneten Dosis X (dX);

   Und daher also die folgenden 4 Schritte zur Berechnung dieses Index, insbesondere:

   (i) Die Bestimmung der Meteorologie, die Einfluss hat auf die Kinetik der Konzentrationen an mineralischem Stickstoff (Nm) im oberen Teil der ungesättigten Zone des Bodens indem aus den monatlichen klimatischen Mittelwerten ein täglicher Niederschlag stochastisch generiert wird, dieser obere Teil der ungesättigten Zone ist begrenzt auf eine Tiefe von 0 bis 50, genauer gesagt von 0 bis 40 cm, und noch besser von 0 bis 30 cm, durch Modellierung der Kinetiken des mineralischen Stickstoffs einschließlich der stochastischen Generierung der Regenmessung ausgehend von den monatlichen klimatischen Mittelwerten und so die Häufigkeit und Menge von Niederschlagsereignissen erzeugt, ausgehend von einer Serie von monatlichen Statistiken wie den mittleren täglichen minimalen und maximalen Lufttemperaturen am Boden, ihre Standardabweichungen, die kumulierte Niederschlagsmenge für den Monat sowie die Anzahl der Regentage im Monat, Übergangswahrscheinlichkeiten und ein bestimmter Index der Gewitterintensität;

(ii) das Überwachen der Milli-Molarität (mM) der Bodenlösung im Zeitverlauf, günstigerweise nach dem Vergraben der RCsol, auf Grundlage der Zahl der Tage nach dem Vergraben (jpe) und berechnet auf Grundlage des Gehalts an Nitraten ($NO_3^-$) und Ammonium ($NH_4^+$) im Boden, seines Schüttgewichts (DA; $g/cm^3$), der Tiefe (Z; cm) des Mutterbodens, dieser sei hier von 0 bis 30 cm, sowie der Wassergehalt, [$H_2O$], dieses Mutterbodens, oder noch genauer nach der Gleichung;

$$\frac{([NO_3]/PM_{NO3}/DA_Z) \ / \ (Z/10)) + ([NH_4]/PM_{NH4}/DA_Z) \ / \ (Z/10))}{[H_2O]/100}$$

und dies noch einmal unter (1) der Generierung des täglichen Wetters mithilfe der stochastischen Generierung, die die mittleren monatlichen klimatischen Werte in Tageswerte umrechnen kann, und (2) der Berechnung der Konzentration an Nm der Bodenlösung in Tages-Inkrementen mithilfe der obengenannten Meteorologie und Analysen des mineralischen Stickstoffs des herbstlichen Bodens mithilfe bekannter dynamischer Modelle der Prozesse Boden/Pflanze, oder auch eventueller *Meta-Modelle*, die Multi-Variablen-Gleichungen umfassen, die die wichtigsten der vorgenannten bodenklimatischen Variablen enthalten;

(iii) die Quantifizierung des Grades der negatvien Rückkopplung des Nm der Diazotrophie (Nif) der Bakterienflora des Bodens im Vergleich zu ihrer maximalen Aktivität entsprechend der obengenannten mM der Nm der Bodenlösung, eine Reduktion (negative Rückkopplung) einfach und günstigerweise beschrieben durch eine Funktion mit negativer Potenz, der Art a·[mM-N]$^{-b}$, die vorhergehende und/oder publizierte Daten umfasst, selbst wenn diese Parametrisierung im Lauf der Zeit feinabgestimmt werden muss, indem (1) diese Konzentration (mM) an mineralischem Stickstoff (Nm) der Bodenlösung in einer empirischen Gleichung angewendet wird, die die abnehmende Aktivität der Diazotrophie aufgrund eines Anstiegs der umgebenden Milli-Molarität von Nm beschreibt, um so einen maximalen Prozentsatz der diazotrophen Aktivität, Nif, zu erhalten, wenn die besagte Milli-Molarität Null ist;

(iv) die Integration über den Zeitverlauf (jpe), am besten durch Summierung, der täglichen Inkremente (jpe) des Bruchs, günstigerweise als Prozentsatz ausgedrückt, der diazotrophen Aktivität (Nif) der Bakterienflora des Bodens im Vergleich zu seinem obengenannten Maximum, wenn die Milli-Molarität des mineralischen Stickstoffs der Bodenlösung mM-Nm, ≈ 0,00 (d.h. % $NIF_{max}$), und dies über einen Zeitraum von mehreren Monaten einschließlich einer Anzahl von Tagen, die nach dem Einackern der verschiedenen Überreste der Kultur (Cellulosepflanzen) in den Boden (RCsol) liegen, (jpe) wo der IDM also hier die Summe, die Kumulation, dieser Prozentsätze im Verhältnis zu den Nif-Maxima (% Nif Max) ist, er darf im Prinzip 100 % nicht überschreiten, während der letzte Wert des IDM von der Periode der Integration (der Kumulierung) abhängt.

2. Methode zur Diagnose des diazotrophen Zustands von Ackerböden und für die Empfehlung der Düngung der Kultur entsprechend des vorhergehenden Patentanspruchs, **dadurch gekennzeichnet, dass** die Bestimmung der Meteorologie, die die Kinetik des mineralischen Stickstoffs (Nm) in im oberen Bereich der ungesättigten Zone des Bodens beeinflusst, durch die Erstellung der Regenmessung ausgehend von den monatlichen klimatischen Mittelwerten wie folgt ausgeführt werden kann:

➤ Entnahme einer Bodenprobe vor der Aussaat der Winterkultur, günstigerweise in Anwesenheit des RCsol der vorhergehenden Kultur, vor allen wenn diese RCsol (azoto)bakterisiert und eingeackert sind;

➤ Analyse dieser Bodenprobe auf ihren Gehalt an mineralischem Stickstoff (Nm) und einschließlich der Werte für den Wassergehalt (eg. % des Bodens, oder auch mm pro Hektar), die Tiefe (eg. cm) der beprobten Humusschicht, sowie sein Schüttgewicht (eg. $g/cm^3$);

➤ monatliches Erheben der Klimatologie der nächstgelegenen meteorologischen Station, inklusive der mittleren monatlichen Minimal- und Maximaltemperaturen, den kumulierten monatlichen Regenmengen und günstigerweise der Anzahl der Regentage jedes Monats;

➤ Generierung der täglichen Meteorologie ausgehend von der zuvorgenannten monatlichen Klimatologie;

3. Methode zur Diagnose des diazotrophen Zustands von Ackerböden und für die Empfehlung der Düngung der Kultur entsprechend des vorhergehenden Patentanspruchs, **dadurch gekennzeichnet, dass** die Generierung der Tages-Meteorologie mithilfe eines bewährten stochastischen Generators gemacht wird, der die monatlichen klimatischen Mittelwerte entsprechend in Tages-Meteorologie umwandeln kann;

4. Methode zur Diagnose des diazotrophen Zustands von Ackerböden und für die Empfehlung der Düngung der Kultur entsprechend eines jeglichen der vorhergehenden Patentansprüche, **dadurch gekennzeichnet, dass** die Verfolgung der Milli-Molarität (mM) der Bodenlösung im Zeitverlauf, günstigerweise folgend auf das Vergraben der Überreste von Cellulosepflanzen im Boden (RCsol), in Abhängigkeit der Anzahl der Tage nach dem Vergraben (jpe) wie folgt ausgeführt werden kann: Berechnung der täglichen Konzentration der Bodenlösung mithilfe der obengenannten Meteorologie und Analysen des mineralischen Stickstoffs der herbstlichen Erde.

5. Methode zur Diagnose des diazotrophen Zustands von Ackerböden und für die Empfehlung der Düngung der Kultur entsprechend des vorhergehenden Patentanspruchs, **dadurch gekennzeichnet, dass** die Berechnung der Tageswerte der Konzentrationen der Bodenlösung mihilfe der obengenannten Meteorologie und Analysen des mineralischen Stickstoffs in der herbstlichen Erde gemacht wird mithilfe von computerisierten Modellen der Prozesse Boden/Pflanze, oder auch mithilfe von "Meta-Modellen", die mithilfe dieser Anwendungen und bestehend aus der Zuhilfenahme von multi-variablen Gleichungen, die die vorgenannten Variablen enthalten (d.h. Gehalte an Nm in den Böden, kumulierte Regenmessungen, Ton-Gehalte der Böden, etc.) entwickelt wurden.

6. Methode zur Diagnose des diazotrophen Zustands von Ackerböden und für die Empfehlung der Düngung der Kultur entsprechend eines jeglichen der vorhergehenden Patentansprüche, **dadurch gekennzeichnet, dass** die Quantifizierung des Grades der negativen Rückkopplung durch Nm der Diazotrophie (Nif) der Bakterienflora des Bodens in Bezug auf ihre maximale Aktivität abhängig von der zuvorgenannten mM des Nm der Bodenlösung wie folgt ausgeführt werden kann; die Anwendung dieser Konzentration, dieser Milli-Molarität (mM), an mineralischem Stickstoff (Nm) der Bodenlösung in einer empirischen Gleichung, die die abnehmende Aktivität der Diazotrophie als Reaktion auf einen Anstieg der umgebenden Milli-Molarität des mineralischen Stickstoffs beschreibt; so erhält man einen Prozentsatz der diazotrophen Aktivität, Nif, wenn die besagte Milli-Molarität Null ist;

7. Methode zur Diagnose des diazotrophen Zustands von Ackerböden und für die Empfehlung der Düngung der Kultur entsprechend eines jeglichen der vorhergehenden Patentansprüche, **dadurch gekennzeichnet, dass** die Integration über die zeit (jpe), vorteilhafterweise und zum Beispiel durch Summierung, von Tages-Inkrementen (jpe) des Bruchs, günstigerweise als Prozentsatz ausgedrückt, der diazotrophen Aktivität (Nif) der Bakterienflora des Bodens in Bezug auf sein Maximum wenn die Milli-Molarität des mineralischen Stickstoffs in der Bodenlösung, mM-Nm, $\approx$ 0,00 (d.h. %NIF$_{max}$) wie folgt ausgeführt werden kann; Bildung der Summe einer bestimmten Zeitperiode, genannt "zwischen-Kulturen", dieser Prozentsätze, %NIF$_{max}$, nach dem herbstlichen Einackern der Reste der Cellulosepflanzen, vorzugsweise (azoto)bakterisiert;

8. Methode zur Diagnose des diazotrophen Zustands von Ackerböden und für die Empfehlung der Düngung der Kultur entsprechend des vorhergehenden Patentanspruchs, **dadurch gekennzeichnet, dass** die Summe über eine bestimmte Zeitperiode, genannt "zwischen-Kulturen", dieser Prozentsätze, %NIF$_{max}$, nach dem herbstlichen Einackern der Reste der Cellulosepflanzen, vorzugsweise (azoto)bakterisiert, *Diazoto-Molarer Index,* oder IDM, genannt wird.

9. Methode zur Diagnose des diazotrophen Zustands von Ackerböden und für die Empfehlung der Düngung der Kultur entsprechend der zwei vorhergehenden Patentansprüche, **dadurch gekennzeichnet, dass** die Summe über eine bestimmte Zeitperiode, genannt "zwischen-Kulturen", dieser Prozentsätze, %NIF$_{max}$, nach dem herbstlichen Einackern der Reste der Cellulosepflanzen, vorzugsweise (azoto)bakterisiert, eine Zeitperiode nach dem Einackern umfasst, von RCsol von Anfang Oktober bis Ende Feber/Anfang März oder von RCsol zum sogenannten "Winterende", gezählt in Tagen nach dem Einackern (jpe).

10. Methode zur Diagnose des diazotrophen Zustands von Ackerböden und für die Empfehlung der Düngung der Kultur entsprechend eines jeglichen der vorhergehenden Patentansprüche, **dadurch gekennzeichnet, dass** die Empfehlung betreffend der Dosierung der zuvor berechneten Dosis X (dX) ausgeführt wird auf Basis der Diagnose des diazotrophen Zustands des Bodens, insofern als die höchsten IDM Anzeiger für eine Nif-Aktivität von Bakterienpopulationen im Boden sind, die groß genug sind, um während der zwischen-Kulturen Zeitperiode fähig zu sein, eine Stickstoff-Reserve aus diazotropher Quelle sicherzustellen, indem Mengen von mineralisierbarem Stickstoff freigesetzt werden, zum Ersetzen von Stickstoff-Einheiten (kg-N von Dünger N/ha), die andernfalls via dX in den Boden gebracht würden, somit kann die dX rationiert werden auf die Höhe von etwa zwanzig Einheiten (kg- N/ha).

**Claims**

1. **Method for the diagnosis of the diazotrophic state of arable soils and for the recommendation of fertilization of an in-situ field crop** by rationing a dose X (dX) of nitrogen fertilizer (N) previously calculated comprising ;

   ➢ an autumn reading of the mineral nitrogen content (Nm) of the surface soils by sampling, without having to sample the entire rooting and/or vadose zone, (1) by taking a sample of soil before sowing the winter crop, (2) by analysing this sample for its mineral nitrogen content (Nm), and (3) by obtaining the monthly climatological readings including the average monthly minimum temperatures and the monthly accumulations of rainfall,

   ➢ the calculation of an integral index herein called the *diazoto-molar index* (IDM) comprised of a numerical sum revealing the incidence of post-burial time units for soil borne cellulosic crop residues (RCsol) more of less conducive as the case may be for a maximum of diazotrophic activity (Nif) of soil bacteria by (1) the summing over a certain period - the interculture - these percentages,% NIFmax, after the fall burial of the said RCsol, and (2) by carrying out a diagnosis in view of fertilizer recommendations, the highest IDM thus revealing an Nif activity throughout the interculture period producing a nitrogen reserve of diazotrophic origin which can release appreciable quantities of mineralizable nitrogen and replace a certain amount of N-fertilizer normally (otherwise) supplied via the dX as well as a recommendation according to the determined IDM pertaining to the rationing of a dose X (dX) as previously calculated ;

   and therefore also the following four steps for the calculation of this index, namely ;

   (i) the determination of the meteorology affecting the kinetics of the mineral nitrogen (Nm) concentrations in the upper section of the vadose zone of the soil by stochastic generation of daily rainfall from monthly climatic averages, this upper section of the zone vadose being limited to a depth of 0 to 50, more particularly 0 to 40 cm, and advantageously from 0 to 30 cm, by modelling the kinetics of mineral nitrogen comprising the stochastic generation of rainfall from the aforementioned monthly climatic averages thus generating the incidence and quantity of rainfall events from a series of monthly statistics such as the average of the minimum and maximum daily air temperatures on the ground, their standard deviations, the cumulative rainfall for the month, as well as that the number of rainy days in the month, the probabilities of transition, and a certain index of intensity of thunderstorms ;
   (ii) monitoring the Nm milli - molarity (mM) of the soil solution over time, advantageously following the burial of the RCsol, as a function of the number of post-burial days (jpe) and calculated as a function of soil nitrates ($NO_3^-$) and ammonium ($NH_4^+$) concentrations, topsoil bulk density (DA; g / cm3) and depth (Z; cm), i.e. 0 to 30 cm in depth, as well as topsoil water content [$H_2O$], or more precisely according to the equation,

$$\frac{([NO_3]/PM_{NO3}/DA_Z) / (Z/10)) + ([NH_4]/PM_{NH4}/DA_Z) / (Z/10))}{[H_2O]/100}$$

   and this again by (1) generating the daily meteorology using stochastic generators capable of converting these monthly average climatic values into daily meteorology, and (2) by calculating in daily increments the Nm concentration of the soil solution using the aforementioned meteorology and analyses of the mineral nitrogen (Nm) of the autumnal soil using recognized dynamic models of soil/plant processes, or even possible metamodels comprising multivariate equations using the most determinants of the above pedoclimatic variables;
   (iii) the quantification of the degree of Nm's negative feedback of the diazotrophic activity (Nif) of the soil's bacterial flora as compared to its maximum activity as a function of the aforesaid Nm mM of the soil solution, reduction (negative feedback) easily and advantageously described by a negative power function of the type a · $[mM-N]^{-b}$ comprising prior and/or published data, even if this setting is fine-tuned thereafter, by (1) inserting this mineral nitrogen (Nm) concentration (mM) by of the soil solution in an empirical equation describing this decreasing diazotrophic activity in response to an increase in the ambient Nm milli-molarity (mM) so as to thereby obtain a percentage of the maximal diazotrophic activity, Nif, when the said Nm milli-molarity is zero;
   (iv) integration over time (jpe), advantageously by summation, of daily increments (jpe) of the fraction, advantageously expressed as a percentage, of the soil's bacterial flora diazotrophic activity (Nif) relative to its aforesaid maximum when the milli-molarity of the mineral nitrogen of the soil solution, mM-Nm, ≈ 0.00 (i.e. %NIFmax), and this over a period in the order of several months comprising a certain number of days post-burial (jpe) of the various (cellulosic) soil borne crop residues (RCsol), the IDM therefore here being the sum, the accumulation, of these percentages compared to the maximum Nif (%Nif Max) in principle not exceeding 100%, whereas the ultimate value of IDM will depend on the period of integration (accumulation).

2. **Method for the diagnosis of the diazotrophic state of arable soils and for the recommendation of fertilization of an in-situ field crop** according to the preceding claim **characterized in that** the determination of the meteorology affecting the kinetics of mineral nitrogen (Nm) in the upper slice of the soil vadose zone by generation of rainfall from monthly climatic averages can be performed as follows ;

> ➢ take any soil sample before sowing the winter crop, advantageously in the presence of RCsol from the previous crop, especially if these RCsol are (azoto)bacteria-treated and buried ;

> ➢ analyze the said sample for its mineral nitrogen content (Nm), including values for the water content (eg.% Of the soil, or even mm per hectare), the depth (eg. Cm) of the sampled topsoil, as well as its bulk density (eg. g / cm3) ;

> ➢ record monthly the climatology of the nearest meteorological station, including the monthly average minimum temperatures, the monthly cumulative rainfall, and advantageously the number of rainy days for each month ;

> ➢ generate the daily meteorology from the aforementioned monthly climatology.

3. **Method for the diagnosis of the diazotrophic state of arable soils and for the recommendation of fertilization of an in-situ field crop** according to the preceding claim **characterized in that** the generation of the daily meteorology is done using a proven stochastic generator which can in this sense convert these monthly average climatic values into daily meteorology.

4. **Method for the diagnosis of the diazotrophic state of arable soils and for the recommendation of fertilization of an in-situ field crop** according to any one of the preceding claims **characterized in that** the monitoring of the Nm milli-molarity (mM) of the soil solution over time, advantageously following the burial of cellulosic soil borne crop residues (RCsol) as a function of the number of days post-burial (jpe) can be carried out as follows; calculate daily the concentration of the soil solution as affected by the above meteorology and analyses of the mineral nitrogen content of the soil in the fall.

5. **Method for the diagnosis of the diazotrophic state of arable soils and for the recommendation of fertilization of an in-situ field crop** according to the preceding claim **characterized in that** the daily calculation of the Nm concentrations of the soil solution using the aforesaid meteorology and analyses of the mineral nitrogen of the soil in the fall is done using computerized soil/plant process models, or again *meta-models* developed using these tools and applications thus summarizing multivariable equations comprising the aforesaid variables (i.e. Nm soil contents, rainfall, soil clay contents, etc.).

6. **Method for the diagnosis of the diazotrophic state of arable soils and for the recommendation of fertilization of an in-situ field crop** according to any one of the preceding claims, **characterized in that** the quantification of the degree of negative feedback per unit of Nm affecting the diazotrophic activity (Nif) of the soil's bacterial flora as compared to its maximum as a function of the aforesaid Nm mM of the soil solution can be carried out as follows ; insert this reading of the mineral nitrogen (Nm) milli-molarity (mM) reading of the soil solution into an empirical equation describing the decreasing diazotrophic activity in response to an increase in the ambient Nm milli-molarity ; a percentage (%) of the maximum diazotrophic activity - Nif - is thus obtained when the said milli-molarity is zero.

7. **Method for the diagnosis of the diazotrophic state of arable soils and for the recommendation of fertilization of an in-situ field crop** according to any one of the preceding claims, **characterized in that** the integration through time, advantageously and as an example by summation of daily increments (jpe) of the fraction, advantageously expressed as a percentage (%) of the diazotrophic activity (Nif) of the soil's bacterial flora as compared to its maximum when the Nm milli-molarity of the soil solution (mM-Nm) $\approx 0.00$ (i.e. %$NIF_{max}$) can be carried out as follows ; add up over a certain period, herein called *interculture*, these percentages, %$NIF_{max}$, after the autumn burying of the cellulosic culture residues, advantageously (azoto)bacteria-treated.

8. **Method for the diagnosis of the diazotrophic state of arable soils and for the recommendation of fertilization of an in-situ field crop** according to the preceding claim, **characterized in that** the sum over a certain period, herein called *interculture*, of these NIFmax percentages following the autumn (fall) burying of cellulosic crop residues, advantageously (azoto)bacteria-treated is called the *diazoto-molar index*, or IDM.

9. **Method for the diagnosis of the diazotrophic state of arable soils and for the recommendation of fertilization of an in-situ field crop** according to the two preceding claims, **characterized in that** the sum over a certain period herein called *interculture* of these NIFmax percentages (%) after the fall (autumn) burying of these advantageously (azoto)bacteria-treated cellulosic crop residues includes a RCsol post-burial period in jpe's stretching from October to the end of February or early March, or again to the simply said *winter's end* (*sortie d'hiver,* fr).

10. **Method for the diagnosis of the diazotrophic state of arable soils and for the recommendation of fertilization of an in-situ field crop** according to any one of the preceding claims, **characterized in that** the recommended rationing of the previously calculated dose X (dX) of nitrogen fertilizer is carried out on the basis of the diagnosed diazotrophic state of the soil, in the sense that the highest IDM's are indicative of a fairly important Nif activity of the soil's bacterial populations throughout the interculture procuring a stock of nitrogen of diazotrophic origin and releasing quantities of mineralizable nitrogen in substitution of N-fertilizers (kg N-fertilizer / ha) otherwise applied to the soil via dX thus rationed by about twenty units (kg-N / ha) or so.

FIGURE 1 :

FIGURE 2 :

**data Laane et al. 1980 (figure 1) et Hartmann et al. 1986 (figures 1A, B et C)**

$y = 35{,}981x^{-0{,}2493}$

$R^2 = 0{,}836$

FIGURE 3 :

**FIGURE 4** :

FIGURE 5 :

FIGURE 6 :

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2223586 A1

- FR 0900867

**Littérature non-brevet citée dans la description**

- **BINGNER, R. L. ; F.D. THEURER.** *AGNPS,* 2009, HTTP://WWW.ARS.USDA.GOV/RESEARCH/DOCS.HTM?DOCID=5199
- **BINGNER, R. L. ; F. D. THEURER.** AGNPS 98: A SUITE OF WATER QUALITY MODELS FOR WATERSHED USE. *PROCEEDINGS OF THE SEDIMENT: MONITORING, MODELING, AND MANAGING, 7TH FEDERAL INTERAGENCY SEDIMENTATION CONFERENCE, RENO, NV, 25-29 MARCH 2001: SUBCOMMITTEE ON SEDIMENTATION OF THE INTERAGENCY ADVISORY COMMITTEE ON WATER DATA,* 25 Mars 2001, VII-1-VII-8
- CONCEPTUAL BASIS, FORMALISATIONS AND PARAMETERIZATION OF THE STICS CROP MODEL. 2008, 304
- **BRISSON N. ; RUGET F. ; GATE P. ; LORGEOU J. ; NICOULLAUD B. ; TAYOT X. ; PLENET D. ; JEUFFROY M.H. ; BOUTHIER A. ; RIPOCHE D.** STICS: A GENERIC MODEL FOR THE SIMULATION OF CROPS AND THEIR WATER AND NITROGEN BALANCES. II. MODEL VALIDATION FOR WHEAT AND CORN. *AGRONOMIE,* 2002, vol. 22, 69-93
- **CLAUDE, PP ; L FILLION.** EFFET DE L'APPORT D'UN INOCULUM BACTÉRIEN AUX RÉSIDUS DE CULTURE DE MAÏS-GRAIN AU SOL SUR LE RENDEMENT ET LA QUALITÉ DE BLÉS D'HIVER PANIFIABLES EN FRANCE. *AGROSOLUTIONS,* Juin 2004, vol. 15 (1), 23-29
- **COMIFER.** CALCUL DE LA FERTILISATION AZOTÉE : GUIDE MÉTHODOLOGIQUE POUR L'ÉTABLISSEMENT DES PRESCRIPTIONS LOCALES - CULTURES ANNUELLES ET PRAIRIES. Avril 2011
- **DE WIT C.T.** SIMULATION OF ASSIMILATION RESPIRATION AND TRANSPIRATION OF CROPS. *SIMULATION MONOGRAPHS,* 1978, 141
- **HARTMANN, A ; H FU ; RH BURRIS.** REGULATION OF NITROGENASE ACTIVITY BY AMMONIUM CHLORIDE IN AZOSPIRILLUM SPP. *JOURNAL OF BACTERIOLOGY,* Mars 1986, 864-870

- **JEGO G. ; SANCHEZ-PEREZ J.M. ; JUSTES E.** PREDICTING SOIL WATER AND MINERAL NITROGEN CONTENTS WITH THE STICS MODEL FOR ESTIMATING NITRATE LEACHING UNDER AGRICULTURAL FIELDS. *AGRICULTURAL WATER MANAGEMENT,* 2012, vol. 107, 54-65
- **LAANE, C ; W KRONE ; W KONINGS ; H HAAKER ; CVEEGER.** SHORT TERM EFFECT OF AMMONIUM CHLORIDE ON NITROGEN FIXATION BY AZOTOBUCTER VINELANDII AND BY BACTEROIDS OF RHIZOBIUM LEGUMINOSUVUM. *EUR. J. BIOCHEM.,* 1980, vol. 103, 39-46
- **LIU J. ; WILLIAMS J.R. ; WANG X. ; YANG H.** USING MODAWEC TO GENERATE DAILY WEATHER DATA FOR THE EPIC MODEL. *ENVIRONMENTAL MODELLING & SOFTWARE,* 2009, vol. 24 (5), 655-664
- **LIU J. ; FRITZ S. ; VAN WESENBEECK C.F.A. ; FUCHS M. ; OBERSTEINER M. ; YANG H.** A SPATIAL EXPLICIT ASSESSMENT OF CURRENT AND FUTURE HOTSPOTS OF HUNGER IN SUB-SAHARAN AFRICA IN THE CONTEXT OF GLOBAL CHANGE. *GLOBAL AND PLANETARY CHANGE,* 2008, vol. 64 (3-4), 222-235
- **THEURER, F.D. ; R.L. BINGNER ; W. FONTENOT ; S.R. KOLIAN.** PARTNERSHIPS IN DEVELOPING AND IMPLEMENTING AGNPS 98: A SUITE OF WATER QUALITY MODELS FOR WATERSHED USE. *PROCEEDINGS OF THE SIXTH NATIONAL WATERSHED CONFERENCE,* 1999, 10
- **WHISLER J.R. ; ACOCK B. ; BAKER D.N. ; FYE R.E. ; HODGES H.F. ; LAMBERT J.R. ; LEMMON H.E. ; MCKINION J.M. ; REDDY, V.R.** CROP SIMULATION MODELS IN AGRONOMIE SYSTEMS. *ADV. AGRON.,* 1986, vol. 40, 141-208
- **WILLIAMS JR.** THE EROSION PRODUCTIVITY IMPACT CALCULATOR (EPIC) MODEL: A CASE HISTORY. *PHIL. TRANS. R. SOC. LOND.,* 1990, vol. 329, 421-428
- THE EPIC MODEL. **WILLIAMS JR.** COMPUTER MODELS OF WATERSHED HYDROLOGY. WATER RESOURCES PUBLICATIONS, HIGHLANDS RANCH, CO, 1995, 909-1000

- THE APEX MODEL. **WILLIAMS JR ; ARNOLD JG ; SRINIVASAN R.** BRC REPORT NO. 00-06. TEXAS AGRIC. EXPT. STATION. TEXAS AGRIC. EXTEN. SERVICE. TEXAS A&M UNIV, 2000